# FASCICULE DE BREVET EUROPEEN

(11) **EP 1 497 391 B1**
(45) Date de publication et mention de la délivrance du brevet: **11.06.2008**
(21) Numéro de dépôt: 03740619.6
(22) Date de dépôt: 16.04.2003
(51) Int. Cl.: C09J 153/00

(54) **COMPOSITIONS ADHESIVES HYDROPHILES**
HYDROPHILE KLEBSTOFFZUSAMMENSETZUNGEN
NOVEL HYDROPHILIC ADHESIVE COMPOSITIONS

(30) Priorité: 17.04.2002 FR 0204779
(43) Date de publication de la demande: 19.01.2005
(73) Titulaire: LABORATOIRES URGO, 21300 Chenove (FR)
(72) Inventeur: AUGUSTE, Stéphane, 21490 Varois et Chaignot (FR); DESMAISON, Nadège, F-21000 Dijon (FR)
(74) Mandataire: Hubert, Philippe
(86) Numéro de dépôt international: PCT/FR2003/001210
(87) Numéro de publication internationale: WO 2003/087254

(56) Documents cités:
- WO-A-02/22735
- FR-A- 2 815 636

## Description

La présente invention est relative à de nouvelles compositions adhésives thermofusibles hydrophiles caractérisées en ce qu'elles comprennent un élastomère thermoplastique du type copolymère séquencé poly(styrène-oléfine-styrène), un produit tackifiant, un plastifiant liquide, de l'eau et un copolymère amphiphile.

L'invention concerne également l'utilisation de ces nouvelles compositions adhésives hydrophiles en tant qu'adhésifs pour fixer tout type de produit et notamment des produits à des fins médicales, dermatologiques ou cosmétologiques qui viennent au contact de la plaie, de la peau ou des muqueuses.

La réalisation de compositions adhésives à base d'un élastomère thermoplastique du type copolymère séquencé poly(styrène-oléfine-styrène) est connue depuis longtemps.

On réalise ainsi des adhésifs sensibles à la pression à partir de compositions comprenant comme constituants essentiels un copolymère séquencé poly(styrène-oléfine-styrène), un produit tackifiant comme par exemple une résine tackifiante et un plastifiant liquide comme par exemple une huile-plastifiante.

De telles compositions sont ainsi définies dans « Handbook of Pressure Sensitive Adhesive technology » 2ème édition édité par Donatas Satas en 1989, chapitre 13, pages 317 à 359.

Ces adhésifs sont utilisés dans de très nombreuses applications industrielles car ils présentent de bonnes propriétés mécaniques (élasticité, cohésion, adhésivité) que l'on peut moduler en jouant sur la nature (grade, viscosité, polarité, masse molaire) et la proportion des trois constituants essentiels que sont le plastifiant, le produit tackifiant et le copolymère séquencé.

Le principal inconvénient des compositions adhésives connues à ce jour provient de leur nature exclusivement hydrophobe, qui les rend incompatibles avec l'eau ou les produits hydrophiles.

Il est connu de rendre ces formulations plus hydrophiles en y incorporant des charges, comme par exemple des dérivés de cellulose, telle la carboxyméthylcellulose de sodium mais ceci en complique la réalisation et en augmente les coûts de fabrication.

De plus, l'incorporation d'eau à de telles compositions provóque le gonflement de la charge hydrophile et la perte des bonnes propriétés mécaniques de la composition comme son adhésivité et sa cohésion.

Il n'existe donc pas aujourd'hui de composition adhésive stable qui puisse contenir de l'eau même en très faible quantité.

On sait par ailleurs que dans le cadre d'une utilisation sur la peau, la plaie ou les muqueuses, qui constituent des supports spécifiques, un adhésif doit répondre à des exigences complexes, et en particulier il doit présenter un aspect acceptable pour l'utilisateur, ne doit pas entraîner de problème de tolérance lors de son utilisation, ni provoquer de douleur ou laisser des traces au retrait et tout ceci en conservant ses propriétés adhésives, cohésives et élastiques lors de l'utilisation.

De plus, il est souhaitable de pouvoir incorporer dans l'adhésif, qui sert alors de réservoir et de moyen de délivrance, divers composés, comme par exemple des actifs pharmaceutiques, cosmétologiques ou dermatologiques.

Des adhésifs plus particulièrement destinés à être utilisés sur la peau, la plaie ou les muqueuses, ont ainsi été décrits dans les demandes de brevet EP 758 009, EP 723 571 et EP 991 730.

Mais les adhésifs décrits dans ces demandes de brevet présentent encore de nombreux inconvénients et ne permettent pas de résoudre de façon satisfaisante un certain nombre de problèmes.

Ainsi, dans certaines applications qui nécessitent l'adjonction de plastifiants liquides en quantité relativement importante, très souvent des huiles, l'adhésif présente de ce fait un aspect gras ou huileux qui manque d'agrément pour l'utilisateur. En outre, la migration de ces plastifiants peut conduire à l'apparition de tâches ou d'auréoles sur les éléments associés à l'adhésif comme par exemple le support, dans le cas d'un patch, voire même à souiller les vêtements en contact avec ce support.

De plus, en raison de leur nature hydrophobe, les adhésifs connus ont un effet occlusif qui entraîne souvent une macération se traduisant par des problèmes de tolérance lors de leur utilisation. Cet effet occlusif se traduit aussi par l'apparition d'une interface aqueuse qui conduit à une perte de l'adhésion et au décollement corrélatif du produit, notamment en cas de transpiration ou lors de la production d'exudats.

Enfin, en raison de cette nature hydrophobe, il est très difficile, et parfois même impossible, d'incorporer dans ces compositions des actifs hydrophiles, dont l'utilisation peut s'avérer très utile, voire indispensable dans certaines applications, comme par exemple un antiseptique, tel le digluconate de chlorhéxidine, ou des extraits végétaux dans le domaine cosmétique, ou encore des électrolytes dans le cadre de la réalisation d'électrodes.

La présence d'eau dans les produits destinés à être appliqués sur la peau, les muqueuses ou sur une plaie apporte une sensation de fraîcheur, de froid ; permet d'améliorer l'aspect du produit final qui apparaît moins gras ; permet d'hydrater et de ramollir les tissus en formant un film hydratant favorisant l'équilibre hydrophysiologique de la peau, de la plaie ou des muqueuses, en évitant ainsi les problèmes précités de tolérance.

C'est pour cela que dans les domaines médicaux, dermatologiques ou cosmétiques, on utilise de préférence des hydrogels au contact de la peau, la plaie ou les muqueuses. Ces hydrogels contiennent de grandes quantités d'eau souvent de l'ordre de 30 à 80% en poids total par rapport au poids total de l'hydrogel, et des polymères naturels de hauts poids moléculaires, comme par exemple les polysaccharides, en particulier les glucomananes, les galactomànanes, les carraghénanes ; ou des polymères synthétiques souvent réticulés pour assurer la cohésion de l'hydrogel adhésif, comme par exemple les copolymères à base d'acide acrylique 2- acrylamido 2 méthylpropanesulfonique commercialisés par la société LUBRIZOL sous la dénomination AMPS^{®}.

Toutefois, ces hydrogels présentent des propriétés d'adhésion et de cohésion insuffisantes. De ce fait, ils se désintègrent et se décollent facilement, la surface de l'adhésif est souvent altérée ou polluée par contact avec la peau, la plaie ou les muqueuses, et ils ne sont pas généralement repositionnables une fois décollés.

A l'inverse des adhésifs , et en raison de leur nature hydrophile, il est très difficile voire impossible d'incorporer à ces hydrogels des produits lipophiles ou hydrophobes, ce qui conduit par conséquent aux mêmes problèmes de formulation qu'évoqués précédemment.

Enfin, la mise en oeuvre des hydrogels est généralement plus délicate et plus complexe que celle des adhésifs .

Compte tenu de cet état de la technique, il serait donc souhaitable de disposer de nouvelles compositions adhésives, qui cumuleraient les avantages des adhésifs connus, à savoir présenter de bonnes propriétés d'adhésion, de cohésion et d'élasticité ; et les avantages des hydrogels, à savoir présenter un caractère hydrophile et contenir de l'eau, même en très faible quantité.

On disposerait ainsi d'adhésifs dont le caractère hydrophile conduirait à de meilleures performances et ouvrirait leur champ d'utilisation à de nouvelles applications. En particulier, dans le cadre des applications à des fins médicales, cosmétiques, pharmaceutiques ou dermatologiques dans lesquelles il y a contact avec la peau, la plaie ou les muqueuses, on disposerait ainsi d'une composition adhésive, peu agressive, bien tolérée, si nécessaire repositionnable, dans laquelle il serait possible d'incorporer des composés hydrophiles ou lipophiles, d'utilisation agréable, susceptible d'apporter une sensation de fraîcheur, simple à mettre en oeuvre, stable et qui conserve ses propriétés d'adhésion et de cohésion lors de son utilisation.

La présente invention a pour objet de nouvelles compositions adhésives , hydrophiles répondant à ces objectifs et permettant notamment de résoudre le problème d'incompatibilité et d'affinité des adhésifs connus avec l'eau et les produits hydrophiles.

Il a été découvert et ceci constitue le fondement de la présente invention qu'il était possible de rendre hydrophile une composition adhésive à base d'élastomère thermoplastique du type copolymère séquencé poly(styrène-oléfine-styrène) de façon satisfaisante et simple de mise en oeuvre en y incorporant un copolymère amphiphile du type poly(styrène-éthylène-butylène-styrène) greffé avec des groupes hydrophiles. Une telle composition peut incorporer de l'eau ou des produits hydrophiles, sans perte de ses propriétés de base.

Ainsi, selon un premier aspect, la présente demande vise à couvrir une composition adhésive hydrophile caractérisée en ce qu'elle comprend :
- un élastomère thermoplastique, choisi parmi les copolymères séquencés poly(styrène-oléfine-styrène), poly(styrène-oléfine) et leurs mélanges
- un produit tackifiant
- un plastifiant liquide
- de l'eau et
- un copolymère amphiphile à blocs du type ABA comportant deux blocs terminaux thermoplastiques A poly(styrène) et un bloc central élastomère B dans lequel le bloc central B est une séquence poly(éthylène-butylène) comportant des groupes hydrophiles greffés, ledit copolymère ABA pouvant être représenté schématiquement par la structure suivante : 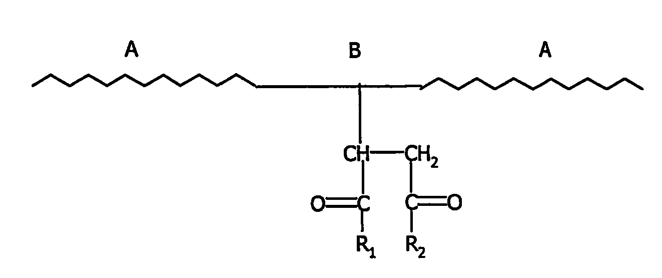
dans laquelle R₁ et R₂ identiques ou différents représentent un groupement hydrophile de masse molaire moyenne inférieure à 10 000, choisi parmi les groupes suivants :

CH₃-O-(CH₂-CH₂-O)ₙ ;

HO-(CH₂-CH₂-O)ₙ ;

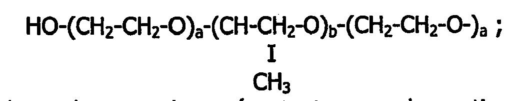

dans lesquels n, a et b représentent un nombre entier.

Les compositions adhésives hydrophiles selon l'invention présentent ainsi une palette de propriétés très large qui rend possible leur exploitation dans de très nombreux domaines et en particulier dans la réalisation de produits destinés à être utilisés au contact de la peau, la plaie ou les muqueuses.

Selon un second aspect, la présente demande vise à couvrir l'utilisation de ces nouvelles compositions adhésives hydrophiles en tant qu'adhésifs pour fixer tout type de produit et notamment des produits à des fins médicales, dermatologiques ou cosmétologiques qui viennent en contact de la plaie, de la peau ou des muqueuses.

La description détaillée qui va suivre des différents constituants de la composition adhésive hydrophile selon l'invention, permettra de mieux comprendre la nature et les applications de cette invention.

### Description de l'invention

Le copolymère amphiphile utilisé dans la réalisation des compositions adhésives hydrophiles selon (Invention est un copolymère à blocs du type ABA comportant deux blocs terminaux thermoplastiques A poly(styrène) et un bloc central élastomère B dans lequel ce bloc central B est une séquence poly(éthylène-butylène) comportant des groupes hydrophiles greffés, ledit copolymère amphiphile ABA pouvant être représenté schématiquement par la structure suivante : 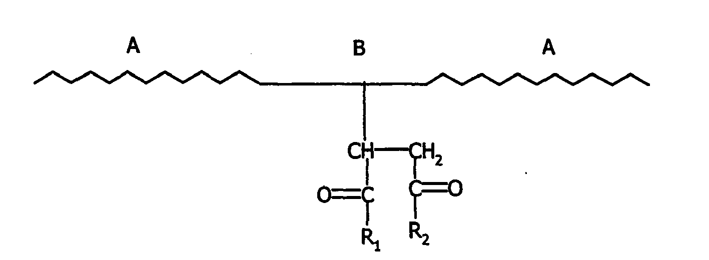 dans laquelle R₁ et R₂ identiques ou différents représentent un groupement hydrophile de masse molaire moyenne inférieure à 10 000, choisi parmi les groupes suivants :

CH₃-O-(CH₂-CH₂-O)ₙ;

HO-(CH₂-CH₂-O)ₙ ;

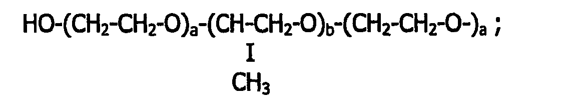

dans lesquels n, a et b représentent un nombre entier.

Avantageusement, dans le cadre de la présente invention, on préférera les copolymères amphiphiles dans lesquels R₁ et R₂ sont identiques.

Parmi ceux-ci on utilisera de préférence les copolymères amphiphiles dans lesquels R₁ et R₂ représentent un groupe CH₃-O-(CH₂-CH₂-O)ₙ, en particulier de masse molaire moyenne comprise entre 1000 et 8000 et tout particulièrement de masse molaire moyenne égale à 2000 (soit n=45).

Ces copolymères amphiphiles sont obtenus par greffage de composés hydrophiles sur un copolymère SEBS particulier.

Ce copolymère particulier comporte des fonctions anhydrides succiniques réparties le long de la chaîne élastomérique poly(éthylène-butylène), obtenues par réaction de l'anhydride maléique sur la séquence poly(éthylène-butylène) et on l'appellera par la suite SEBS maléisé.

Ce copolymère SEBS maléisé, qui sert de base à la réalisation des copolymères amphiphiles selon l'invention, peut être représenté schématiquement par la formule suivante :

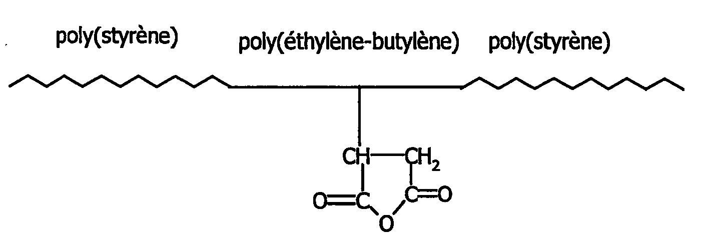

Dans cette formule, on n'a représenté, pour des raisons de simplicité, qu'un seul groupe anhydride succinique sur la séquence poly(éthylène-butylène). Il est bien évident que cette séquence comporte en réalité plusieurs groupes anhydriques succiniques. Cette simplification a également été utilisée pour représenter schématiquement les copolymères amphiphiles utilisés dans le cadre de la présente invention.

En tant que SEBS maléisé on préférera un SEBS maléisé commercialisé par la société SHELL sous la dénomination Kraton G 1901^{®} qui contient 2 % en poids de fonctions anhydrides succiniques fixées sur la chaîne élastomérique et 28 % en poids de polystyrène.

Ce sont ces fonctions anhydrides qui vont servir à greffer les composés hydrophiles par réaction chimique entre le composé hydrophile et l'anhydride ou sa forme acide.

En effet, selon les conditions de stockage et en particulier suivant le degré de séchage de ce SEBS maléisé une partie de ces fonctions anhydrides succiniques peuvent se présenter sous leurs formes acide après ouverture de l'anhydride en présence d'eau. La réaction s'effectue alors aussi entre les fonctions acides et le composé hydrophile.

Les composés hydrophiles qui sont greffés sur le SEBS maléisé sont de 3 types :

### A/ Les polyéthylèneglycols appelés ci-après en abrégé PEG

Ce sont des polymères hydrophiles, hygroscopiques et stables thermiquement. Ils sont utilisés dans de très nombreux domaines industriels. Ils sont bien connus de l'homme de l'art. Ce sont des polymères de courtes chaînes possédant des fonctions hydroxyles aux extrémités. Leur masse molaire moyenne varie de 200 à 20 000.

Leur composition correspond à la structure suivante :
HO-(CH₂-CH₂-O)ₙ-H, dans laquelle n représente un nombre entier.

De tels produits sont par exemple commercialisés par la société Aldrich sous la dénomination poly(éthylèneglycol) suivie de la masse molaire moyenne du PEG considéré, par exemple poly(éthylèneglycol) 2000.

Dans le cadre de la présente invention seuls des copolymères amphiphiles dans lesquels les PEG de masse molaire moyenne inférieure ou égale à environ 10 000 (n ayant donc au maximum une valeur de 230) sont utilisés. En effet, au-delà la réaction de greffage devient difficile voire impossible.

Avantageusement, on utilisera les PEG qui ont une masse molaire moyenne comprise entre 1 000 et 8 000 et en particulier le PEG qui a une masse molaire moyenne de 2 000 (n = 45).

### B/ Les polyéthylèneglycols mono méthyl éther appelés ci-après en abrégé PEGME

Ce sont aussi des polymères de courtes chaînes utilisés comme les PEG dans de très nombreux domaines et bien connus de l'homme de l'art.

Ils ont la structure suivante :
CH₃-O-(CH₂-CH₂-O)ₙ-H, dans laquelle n est un nombre entier, et leur masse molaire moyenne varie de 200 à 20 000.

De tels produits sont par exemple commercialisés par la société Aldrich sous la dénomination poly(éthylèneglycol)méthyl éther suivie de la masse molaire moyenne du PEGME considéré, par exemple poly(éthylèneglycol)méthyléther 2000.

Dans le cadre de la présente invention on utilise des copolymères amphiphiles dans lesquels tout comme pour les PEG, seuls les PEGME de masse molaire moyenne inférieure ou égale à environ 10 000 (n ayant au maximum une valeur de 230) sont utilisés.

Avantageusement, on utilisera les PEGME qui ont une masse molaire moyenne comprise entre 1000 et 8 000 et en particulier le PEGME qui a une masse molaire moyenne de 2 000 (n = 45).

### C/ Les copolymères de polyéthylène-polypropylèneglycol

Ce sont des copolymères très connus que l'on désignera ci-après en abrégé PEO/PPO/PEO.

Ce sont des copolymères triblocs dont la partie centrale est un bloc oxyde de polypropylène et les extrémités des blocs oxyde de polyéthylène qui ont la structure suivante : 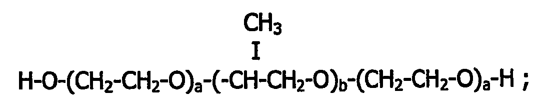 dans laquelle a et b sont des nombres entiers.

Ils sont souvent désignés sous le terme général de poloxamer.

Il existe pour ces produits de très nombreux grades caractérisés par les valeurs de a et b qui définissent leurs masses molaires moyennes. On peut ainsi citer :
- poloxamer 124 : a=12 b=20 masse molaire moyenne comprise entre 2 090 et 2360
- poloxamer 188 : a=80 b=27 masse molaire moyenne comprise entre 7 680 et 9510
- poloxamer 407 : a=101 b=56 masse molaire moyenne comprise entre 9 840 et 14 600.

Ils sont commercialisés par exemple par la société BASF sous la dénomination Pluronic^{®}.

Ici aussi comme précédemment seuls les PEO/PPO/PEO de masse molaire moyenne inférieure ou égale à environ 10 000 seront utilisés.

Dans le cadre de la présente invention on préférera un PEO/PPO/PEO de masse molaire voisine de 2000 comme par exemple le produit commercialisé sous la dénomination poly(éthylèneglycol)-block-poly(propylèneglycol)-block-poly-(éthylèneglycol) 1900 par la société Aldrich, de masse molaire moyenne égale à 1900.

Les copolymères amphiphiles utilisables dans le cadre de la présente invention peuvent être facilement préparés par réaction d'estérification entre les fonctions anhydrides succiniques du SEBS maléisé et les fonctions hydroxyles des PEG, PEGME ou PEO/PPO/PEO utilisés.

La réaction d'un alcool sur une fonction anhydride donne de façon réversible un ester. Dans le cadre de la présente invention, cette estérification peut être représentée par le schéma simplifié suivant :

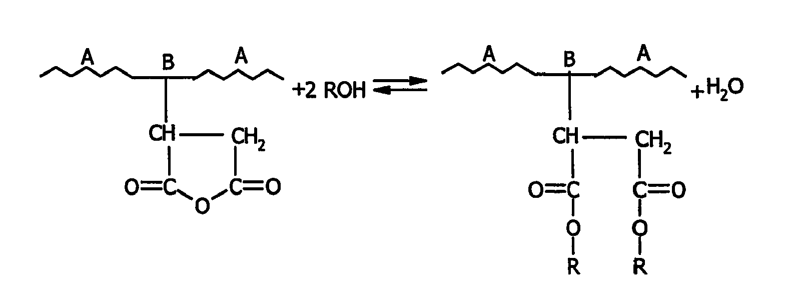

Afin de favoriser la réaction d'estérification on introduit un excès de fonctions hydroxyles par rapport aux fonctions anhydrides. La réaction est avantageusement catalysée par un acide, et l'eau formée est éliminée par distillation azéotropique pour déplacer l'équilibre vers le produit greffé. La réaction est réalisée de préférence sous atmosphère inerte.

Ainsi on prépare lesdits copolymères amphiphiles selon un procédé dans lequel on réalise une réaction d'estérification entre les fonctions anhydrides succiniques portées par la partie poly(éthylène-butylène) d'un copolymère poly(styrène)-poly(éthylène-butylène)-poly(styrène) (SEBS maléisé) et les fonctions hydroxyles d'un composé hydrophile choisi parmi les polyéthylèneglycols, (PEG), les polyéthylèneglycols mono méthyléther (PEGME) et les copolymères de polyéthylène-propylène glycol (PEO/PPO/PEO) de masse molaire moyenne inférieure ou égale à 10 000 ou leurs mélanges, de préférence en présence d'un catalyseur acide, en éliminant l'eau formée et avec un excès de fonctions hydroxyles par rapport aux fonctions anhydrides succiniques du SEBS maléisé.

De façon plus précise le procédé de synthèse est le suivant :
On dissout à chaud (à environ 120 °C température de reflux du solvant) et sous agitation le SEBS maléisé dans un solvant, de préférence le toluène.

On prépare à part une solution d'au moins un composé hydrophile (PEG, PEGME, PEO/PPO/PEO ou leurs mélanges) en chauffant ce(s) dernier(s) à sa(leur) température de fusion sous agitation dans un solvant, de préférence le toluène. On utilise avantageusement un excès de composés hydrophiles. Le nombre de fonctions hydroxyles rapporté au nombre de fonctions anhydrides peut ainsi varier de 2,5 à 20.

A la solution de copolymère SEBS maléisé préalablement obtenue sous agitation et toujours à reflux, on ajoute une quantité catalytique (environ quelques gouttes) d'acide, par exemple d'acide sulfurique, puis la solution de composé(s) hydrophile(s) dans le solvant préparée précédemment.

On agite sous distillation azéotropique ce mélange à reflux durant 30 minutes à 5 heures selon la nature du(des) composé(s) hydrophile(s) jusqu'à réalisation complète de la réaction d'estérification entre les fonctions anhydrides (ou leurs éventuelles formes acides) des groupes succiniques du SEBS maléisé et les fonctions hydroxyles du(des) composé(s) hydrophile(s). L'état d'avancement de la réaction est suivi en utilisant les techniques bien connues de l'homme de l'art, par exemple par spectroscopie infra rouge jusqu'à disparition du pic d'absorption des carbonyls de l'anhydride soit 1785 cm⁻¹.

On précipite alors le mélange réactionnel à chaud à environ 90-100 °C dans un solvant de précipitation adéquat comme par exemple l'éthanol ou un mélange éthanol/eau, ledit solvant de précipitation représentant environ 4 fois le volume de l'ensemble des solvants réactionnels utilisés.

Après filtration, les solvants résiduels sont éliminés du copolymère SEBS amphiphile obtenu, par évaporation à l'étuve sous vide à 40-50 °C.

Il est alors nécessaire de purifier ce dernier pour éliminer le(s) composé(s) hydrophile(s) PEG, PEGME ou PEO/PPO/PEO utilisé(s) en excès encore présent(s).

On redissout donc le polymère amphiphile obtenu sous agitation à environ 90 à 110°C dans le toluène et on reprécipite la solution obtenue dans le même solvant et le même volume que lors de l'étape de précipitation réalisée précédemment à la fin de la synthèse.

De même le copolymère SEBS amphiphile est récupéré par filtration et séché à nouveau à l'étuve sous vide à 40-50°C.

On répète cette étape de purification jusqu'à élimination totale du(des) composé(s) hydrophile(s) en vérifiant selon les techniques connues de l'homme de l'art l'absence du pic de ce(ces) dernier(s) par chromatographie par perméation de gel (GPC).

Dans le cadre de la présente invention on utilisera de préférence ce copolymère amphiphile dans les compositions adhésives à une concentration de l'ordre de 0,05% à 20% en poids par rapport au poids total de la composition.

Selon un mode de réalisation préférée de l'invention on utilisera un copolymère amphiphile à blocs du type ABA comportant deux blocs terminaux thermoplastiques A poly(styrène) et un bloc central élastomère B dans lequel ce bloc central B est une séquence poly(éthylène-butylène) comportant des groupes hydrophiles greffés, ledit copolymère amphiphile ABA pouvant être représenté par la structure suivante 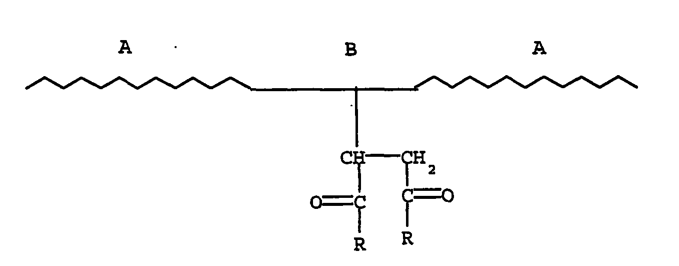 dans laquelle R représente un groupe CH₃-O-(CH₂-CH₂-O)ₙ de masse molaire moyenne égale à 2000 soit n=45.

On préférera tout particulièrement un tel copolymère amphiphile qui a une masse molaire moyenne, mesurée par chromatographie par perméation de gel, de l'ordre de 50 000 daltons.

De préférence, on utilisera alors ce dernier dans les compositions adhésives hydrophiles à une concentration de l'ordre de 0,05 à 20% et en particulier de 0,05 à 5% en poids par rapport au poids total de la composition.

Les élastomères du type copolymères séquencés (styrène-oléfine-styrène) ou copolymères séquencés (styrène-oléfine) qui sont susceptibles d'être utilisés dans le cadre de la présente invention sont ceux habituellement utilisés par l'homme de l'art dans la préparation des adhésifs sensibles à la pression et l'on pourra se reporter à cet égard au document de l'état de la technique mentionné précédemment et en particulier à l'ouvrage de Donatas Satas « Handbook of Pressure Sensitive Technology ».

Ce sont donc soit des copolymères triblocs du type ABA comportant deux blocs terminaux thermoplastiques A styrène et une séquence centrale élastomère B qui est une oléfine, soit des copolymères diblocs du type AB comportant un bloc thermoplastique A styrène et une séquence élastomère B qui est une oléfine. Les séquences B oléfines de ces copolymères peuvent être constituées d'oléfines insaturées comme par exemple isoprène ou butadiène ou d'oléfines saturées comme par exemple éthylène-butylène ou éthylène-propylène.

On pourra, dans le cadre de la présente invention, utiliser l'ensemble de ces produits seuls ou en mélange.

Dans le cas d'un mélange de copolymères triblocs ABA et de copolymères diblocs AB, on pourra employer des mélanges de copolymères triblocs ABA et de copolymères diblocs AB commerciaux déjà disponibles ou réaliser un mélange en toute proportion préalablement choisie à partir de deux produits indépendamment.

Les produits à séquence centrale insaturée sont bien connus de-l'homme de l'art et sont par exemple commercialisés par la société SHELL sous la dénomination KRATON^{®} D. On peut aussi citer pour les copolymères poly(styrène-isoprène-styrène) (en abrégé SIS) les produits commercialisés sous les dénominations KRATON^{®} D 1107 ou KRATON^{®} D 1161 et pour les copolymères poly(styrène-butadiène-styrène) par exemple le produit commercialisé sous la dénomination KRATON^{®} D 1102. D'autres copolymères poly(styrène-isoprène-styrène) sont aussi commercialisés par la société EXXON MOBIL CHEMICAL sous la dénomination VECTOR^{®} comme par exemple le produit commercialisé sous la dénomination VECTOR^{®} 4113. Comme exemples de mélanges commerciaux de copolymères triblocs ABA et diblocs AB dans lesquels B est de l'isoprène, on peut citer le produit commercialisé par la société EXXON MOBIL CHEMICAL sous la dénominationVECTOR^{®} 4114 ou le VECTOR^{®} désigné par le code DPX-565.

Tous ces copolymères à base d'isoprène ou de butadiène présentent généralement une teneur en styrène comprise entre 10 et 52% en poids par rapport au poids total dudit copolymère.

Dans le cadre de la présente invention, on préférera les copolymères séquencés triblocs poly(styrène-isoprène-styrène) ayant une teneur en styrène comprise entre 14 et 30% en poids par rapport au poids dudit SIS. On préférera tout particulièrement comme copolymère séquencé triblocs poly(styrène-isoprène-styrène) le produit commercialisé par la société SHELL sous la dénomination KRATON^{®} D1161 et comme mélange de copolymère séquencé triblocs poly(styrène-isoprène-styrène) et copolymère séquencé diblocs poly(styrène-isoprène) le produit VECTOR^{®} DPX-565 commercialisé par la société EXXON MOBIL Chemical.

Les produits à séquence centrale saturée sont aussi bien connus de l'homme de l'art et sont par exemple commercialisés par la société SHELL sous la dénomination KRATON^{®} G pour les copolymères séquencés poly(styrène-éthylène-butylène-styrène) (en abrégé SEBS) comme par exemple les produits commercialisés sous les dénominations KRATON^{®} G 1651 ou KRATON^{®} G 1654 ou par la société KURARAY sous la dénomination SEPTON^{®} pour les copolymères séquencés poly(styrène-éthylène-propylène-styrène) (en abrégé SEPS).

Comme exemple de mélanges commerciaux de copolymères tribloc-dibloc on peut citer le produit dont la séquence oléfine est éthylène-butylène commercialisé par la société SHELL sous la dénomination KRATON^{®} G 1657.

Comme exemple d'un mélange particulier tribloc-dibloc, que l'on peut utiliser dans le cadre de la présente invention, on peut citer le mélange d'un SEBS tribloc comme le produit commercialisé par la société SHELL sous la dénomination KRATON^{®} G 1651 avec un matériau dibloc poly(styrène-oléfine) comme le poly(styrène-éthylène-propylène) commercialisé par la société SHELL sous la dénomination KRATON^{®} G 1702.

Dans le cadre de la présente invention, on préférera les copolymères triblocs SEBS ou SEPS et en particulier ceux ayant une teneur en styrène comprise entre 25 et 45 % en poids par rapport au poids dudit SEBS. On préférera tout particulièrement le produit commercialisé par la société SHELL sous la dénomination KRATON^{®} G 1651.

De façon générale, on utilisera l'élastomère thermoplastique selon la nature du copolymère séquencé, en une quantité de l'ordre de 2 à 20% en poids par rapport au poids total de la composition. De préférence, on utilisera un élastomère thermoplastique qui a une masse molaire moyenne supérieure à celle du copolymère amphiphile et de préférence de l'ordre de 100 000 daltons. Dans ce cas, on utilisera alors ce dernier de préférence en une quantité de l'ordre de 5 à 15% en poids par rapport au poids total de la composition.

Si nécessaire, on pourra ajouter à ces copolymères séquencés des agents antioxydants. Par agents antioxydants, on entend désigner ici les composés couramment employés par l'homme de l'art pour assurer la stabilité vis-à-vis de l'oxygène, la chaleur, l'ozone et les rayonnements ultra violets des composés utilisés dans la formulation des compositions adhésives, en particulier les résines tackifiantes et les copolymères séquencés. On peut utiliser un ou plusieurs de ces agents antioxydants en association.

On peut citer comme agents antioxydants appropriés les antioxydants phénoliques comme par exemple les produits commercialisés par la société CIBA-GEIGY sous les dénominations IRGANOX^{®} 1010, IRGANOX^{®} 565, IRGANOX^{®} 1076 et des antioxydants soufrés comme par exemple le dibutyldithlocarbamate de zinc commercialisé par la société AKZO sous la dénomination PERKACIT^{®} ZDBC.

Dans le cadre de la présente invention on entend par produit "tackifiant" tout produit qui permet de rendre la composition adhésive.

Etant donné que la composition contient à la fois une phase hydrophobe que l'on peut qualifier de lipophile ou huileuse et une phase aqueuse, on peut donc envisager de la rendre adhésive en incorporant un produit tackifiant dans l'une au moins des deux phases. On pourra ainsi avantageusement employer un produit tackifiant compatible avec la phase huileuse ou un produit tackifiant compatible avec la phase aqueuse, ou encore un ensemble de deux produits tackifiants, un lipophile et un hydrophile, un dans chaque phase.

Dans le cadre de la réalisation de compositions adhésives destinées à être appliquées sur la peau, la plaie ou les muqueuses, la présence d'eau et la possibilité d'utiliser deux types de produits tackifiants très différents offrent une large gamme de solutions pour le réglage, toujours problématique, de la bioadhésion et pour éviter, lors du retrait, l'altération des couches supérieures de l'épiderme ou la pollution, suite à cette desquamation, de la surface de l'adhésif qui empêche souvent de le recoller.

Dans le cadre de la présente invention, on pourra donc utiliser un ou des produits tackifiants dans une large proportion de l'ordre de 1 à 50% en poids par rapport au poids total de la composition en fonction des autres éléments de cette dernière pour obtenir le pouvoir adhésif souhaité pour la composition finale. De préférence, on utilisera un produit tackifiant ou un ensemble de produits tackifiants dans une proportion de 2 à 30% en poids par rapport au poids total de la composition.

Dans le cadre de l'utilisation de 2 produits tackifiants, l'un compatible avec la phase aqueuse et l'autre avec la phase huileuse, on utilisera de préférence l'ensemble de ces derniers dans une proportion totale de l'ordre de 8 à 25% et notamment de 8 à 15% en poids par rapport au poids total de la composition et en particulier une proportion de l'ordre de 2 à 5% en poids de produit tackifiant compatible avec la phase aqueuse et de 8 à 12% en poids de produit tackifiant compatible avec la phase huileuse et pour ce dernier tout particulièrement 10% de polybutène de bas poids moléculaire par rapport au poids total de la composition.

Les produits tackifiants compatibles avec la phase huileuse susceptibles d'être utilisés dans le cadre de la présente invention sont ceux habituellement utilisés par l'homme de l'art dans la préparation des adhésifs sensibles à la pression comprenant des élastomères et en particulier des copolymères séquencés poly (styrène-oléfine-styrène) et l'on pourra se reporter à cet égard au document de l'état de la technique mentionnée précédemment en particulier à l'ouvrage de Donatas Satas.

Dans le cadre de la présente invention, ces produits sont en général choisis parmi les résines tackifiantes, et les polybutènes de bas poids moléculaires ou leurs mélanges.

Parmi les résines tackifiantes qui conviennent selon l'invention, on peut mentionner les résines polyterpènes ou terpènes modifiées, les résines de colophane hydrogénée, les résines de colophane polymérisée, les résines d'esters de colophane, les résines hydrocarbonnées, les mélanges de résine cycliques aromatiques et aliphatiques etc...ou leurs mélanges.

De tels produits sont par exemple commercialisés par la société GOOD YEAR sous la dénomination WINGTACK^{®} comme en particulier la résine de synthèse formée de copolymères en C5/C9 commercialisée sous la dénomination WINGTACK^{®} 86 ou à base de polyterpène synthétique commercialisée sous la dénomination WINGTACK^{®} 10. On peut aussi citer à titre d'exemple les résines commercialisées sous la dénomination KRISTALEX^{®} par la société Herculès comme en particulier la résine à base d'alpha-méthylstyrène KRISTALEX^{®} 3085.

Dans le cadre de la présente invention, on préférera les résines commercialisées par la société EXXON MOBIL CHEMICAL sous la dénomination ESCOREZ^{®} et tout particulièrement la résine de synthèse commercialisée sous la dénomination ESCOREZ^{®} 5300.

Dans le cadre de la présente invention, on utilisera de préférence ces résines dans une proportion de l'ordre de 2 à 30% en poids par rapport au poids total de la composition en fonction du pouvoir adhésif souhaité pour la composition finale. Tout particulièrement, on utilisera une proportion de résines de 5 à 25 % en poids par rapport au poids total de la composition.

Comme polybutènes de bas poids moléculaire utilisables en tant que produit tackifiant de la phase huileuse, on peut citer lesproduits bien connus de l'homme de l'art qui sont par exemple commercialisés sous la dénomination NAPVIS^{®}par la société BP CHIMIE.

Dans le cadre de la présente invention, on préférera tout particulièrement le produit commercialisé sous la dénominationNAPVIS^{®} 10 Ces polybutènes peuvent être utilisés seuls ou en mélange. Ils seront utilisés de préférence dans une proportion de 5 à 30% en poids par rapport au poids total de la composition et tout particulièrement de 8 à 15% en poids.

Les produits tackifiants compatibles avec la phase aqueuse susceptibles d'être utilisés dans le cadre de la présente invention sont habituellement utilisés par l'homme de l'art pour augmenter ou conférer des propriétés de collant en présence d'eau. Ce sont généralement des polymères synthétiques hydrosolubles non réticulés tels les polymères ou copolymères de polyvinylpyrrolidone comme par exemple les polymères de vinylpyrrolidone commercialisés par la société BASF sous la dénomination KOLLIDON^{®} tel le produit KOLLIDON^{®} 30, les polymères ou copolymères d'alcool polyvinylique, les polymères acryliques comme en particulier les polyacrylates commercialisés par la société BF GOODRICH sous la dénomination CARBOPOL^{®} ou les polymères d'éther de polyvinyle hydrosolubles par exemple le produit commercialisé par la société BASF sous la dénomination LUTONAL^{®} M40.

Dans le cadre de la présente invention on utilisera de préférence ces polymères hydrosolubles dans une proportion de l'ordre de 1 à 30% en poids par rapport au poids total de la composition en fonction du pouvoir adhésif souhaité pour la composition finale. Tout particulièrement, on utilisera une proportion de polymères hydrosolubles de l'ordre de 2 à 20 % en poids par rapport au poids total de la composition.

Dans le cadre de la présente invention on entend par "plastifiant liquide" les plastifiants qui sont habituellement utilisés par l'homme de l'art pour la préparation des adhésifs sensibles à la pression comprenant des élastomères thermoplastiques en particulier du type copolymères séquencés poly(styrène-oléfine-styrène) et qui sont des produits qui permettent d'améliorer leurs propriétés d'étirement, de souplesse, d'extrudabilité ou de mise en oeuvre et l'on pourra se reporter à cet égard aux documents de l'état de la technique mentionnés précédemment.

Ces plastifiants liquides sont des composés compatibles avec la séquence centrale oléfine des copolymères séquencés utilisés. On utilise très souvent comme plastifiant liquide des huiles plastifiantes, et en particulier des huiles minérales qui sont formées de composés de nature paraffinique, naphténique ou aromatique ou de leurs mélanges dans des proportions variables.

On peut ainsi citer comme exemple d'huiles minérales les produits commercialisés par la société SHELL sous la dénomination ONDINA^{®} et RISELLA^{®} pour les mélanges à base de composés naphténiques et paraffiniques ou sous la dénomination CATENEX^{®} pour les mélanges à base de composés naphténiques, aromatiques et paraffiniques.

Dans le cadre de la présente invention on préférera les huiles de paraffine et en particulier l'huile commercialisée par la société SHELL sous la dénomination ONDINA^{®} 15.

On peut aussi utiliser comme plastifiant liquide non pas une huile plastifiante mais des produits de synthèse à base de mélanges liquides d'hydrocarbures saturés comme par exemple les produits commercialisés par la société TOTAL sous la dénomination GEMSEAL^{®} comme en particulier le produit GEMSEAL^{®} 60 qui est un mélange isoparaffinique issu d'une coupe pétrolière totalement hydrogénée.

Dans le cadre de la réalisation d'une composition adhésive hydrophile selon l'invention, on utilisera de préférence une concentration en plastifiant liquide de l'ordre de 20 à 90% en poids par rapport au poids total de la composition et tout particulièrementde 30 à 75% en poids par rapport au poids total de la composition adhésive hydrophile.

La composition adhésive hydrophile selon l'invention comprend enfin de l'eau. On pourra utiliser tout type d'eau suivant les domaines d'applications envisagés comme par exemple de l'eau de source, de l'eau du robinet, de l'eau déminéralisée, purifiée, déionisée ou stérilisée.

On pourra bien entendu incorporer avec cette eau tout adjuvant utile pour conserver au cours du temps ces propriétés de pureté ou de stérilité.

De même suivant l'application envisagée, on pourra introduire de très faibles quantités d'eau de l'ordre de 1% en poids par rapport au poids total de la composition ou de grandes quantités allant jusqu'à 60% ou plus en poids par rapport au poids total de la composition.

Dans le cadre de la présente invention on préfère des compositions adhésives qui comprennent de l'ordre de 1 à 50% en poids d'eau et tout particulièrement de 10 à 45% en poids par rapport au poids total de la composition.

Une composition actuellement préférée de l'invention comprend :
a. 2 à 20 parties en poids d'un élastomère thermoplastique de masse molaire moyenne supérieure ou égale à 100 000 daltons
b. 30 à 75 parties en poids de plastifiant liquide
c. 2 à 30 parties en poids de produit tackifiant
d. 1 à 45 parties en poids d'eau et
e. 0,05 à 5 parties en poids d'un copolymère amphiphile à blocs du type ABA comportant deux blocs terminaux thermoplastiques A poly(styrène) et un bloc central élastomère B dans lequel le bloc central B est une séquence poly(éthylène-butylène) comportant des groupes hydrophiles greffés, ledit copolymère amphiphile ABA pouvant être représenté schématiquement par la structure suivante : 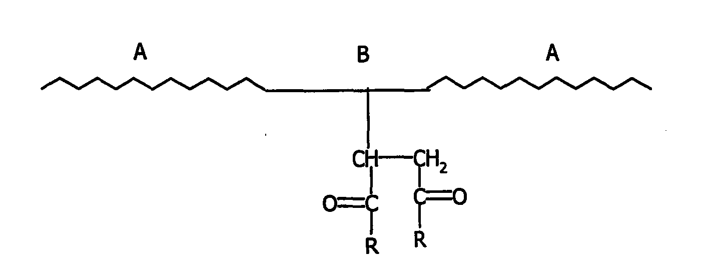
dans laquelle R représente un groupe CH₃-O-(CH₂-CH₂-O)ₙ de masse molaire moyenne égale à 2000 soit n=45 et qui a une masse molaire moyenne mesurée par chromatographie par perméation de gel de 50 000 daltons.

Parmi les compositions adhésives hydrophiles englobées dans cette dernière, on préférera tout particulièrement les compositions suivantes :
Une composition qui comprend :
   a. 2 à 20 parties en poids d'un élastomère thermoplastique de masse molaire moyenne supérieure ou égale à 100 000 daltons
   b. 30 à 75 parties en poids de plastifiant liquide
   c. 5 à 25 parties en poids de résine tackifiante
   d. 10 à 40 parties en poids d'eau et
   e. 0,05 à 5 parties en poids d'un copolymère amphiphile à blocs du type ABA comportant deux blocs terminaux thermoplastiques A poly(styrène) et un bloc central élastomère B dans lequel le bloc central B est une séquence poly(éthylène-butylène) comportant des groupes hydrophiles greffés, ledit copolymère amphiphile ABA pouvant être représenté schématiquement par la structure suivante : 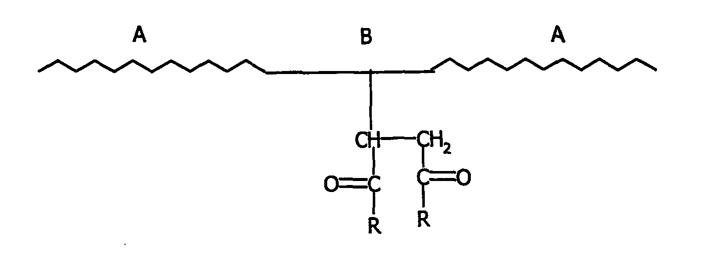
   dans laquelle R représente un groupe CH₃-O-(CH₂-CH₂-O)ₙ de masse molaire moyenne égale à 2000 soit n=45 et qui a une masse molaire moyenne mesurée par chromatographie par perméation de gel de 50 000 daltons.
Une composition qui comprend :
   a. 2 à 20 parties en poids d'un élastomère thermoplastique de masse molaire moyenne supérieure ou égale à 100 000 daltons
   b. 30 à 75 parties en poids de plastifiant liquide
   c. 8 à 15 parties en poids de polybutène de bas poids moléculaire
   d. 10 à 40 parties en poids d'eau et
   e. 0,05 à 5 parties en poids d'un copolymère amphiphile à blocs du type ABA comportant deux blocs terminaux thermoplastiques A poly(styrène) et un bloc central élastomère B dans lequel le bloc central B est une séquence poly(éthylène-butylène) comportant des groupes hydrophiles greffés, ledit copolymère amphiphile ABA pouvant être représenté schématiquement par la structure suivante : 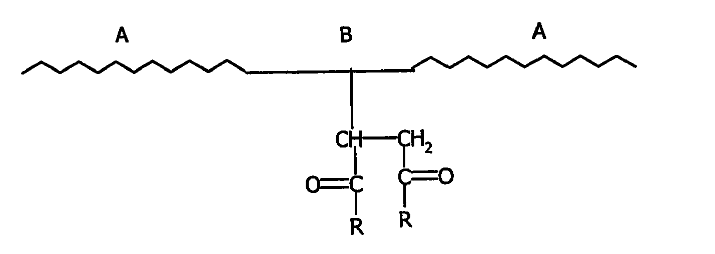
   dans laquelle R représente un groupe CH₃-O-(CH₂-CH₂-O)ₙ de masse molaire moyenne égale à 2000 soit n=45 et qui a une masse molaire moyenne mesurée par chromatographie par perméation de gel de 50 000 daltons.
Une composition qui comprend :
   a. 2 à 20 parties en poids d'un élastomère thermoplastique de masse molaire moyenne supérieure ou égale à 100 000 daltons
   b. 30 à 75 parties en poids de plastifiant liquide
   c. 2 à 20 parties en poids de polymère hydrosoluble
   d. 10 à 40 parties en poids d'eau et
   e. 0,05 à 5 parties en poids d'un copolymère amphiphile à blocs du type ABA comportant deux blocs terminaux thermoplastiques A poly(styrène) et un bloc central élastomère B dans lequel le bloc central B est une séquence poly(éthylène-butylène) comportant des groupes hydrophiles greffés, ledit copolymère amphiphile ABA pouvant être représenté schématiquement par la structure suivante : 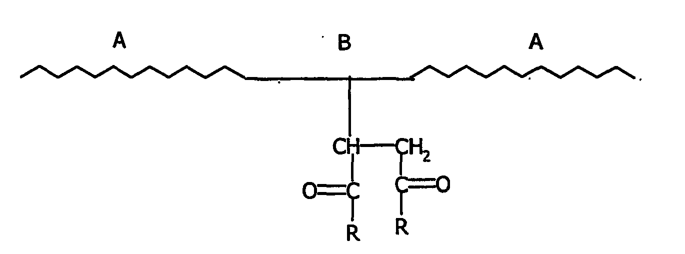
   dans laquelle R représente un groupe CH₃-O-(CH₂-CH₂-O)ₙ de masse molaire moyenne égale à 2000 soit n=45 et qui a une masse molaire moyenne mesurée par chromatographie par perméation de gel de 50 000 daltons.
Une composition qui comprend :
   a. 2 à 20 parties en poids d'un élastomère thermoplastique de masse molaire moyenne supérieure ou égale à 100 000 daltons
   b. 30 à 75 parties en poids de plastifiant liquide
   c. 8 à 15 parties en poids d'un ensemble de produits tackifiants constitué d'un polybutène de bas poids moléculaire et d'un polymère hydrosoluble et tout particulièrement de 10 parties en poids de polybutène de bas poids moléculaire et de 2 à 5 parties de polymère de vinylpyrrolidone.
   d. 10 à 40 parties en poids d'eau et
   e. 0,05 à 5 parties en poids d'un copolymère amphiphile à blocs du type ABA comportant deux blocs terminaux thermoplastiques A poly(styrène) et un bloc central élastomère B dans lequel le bloc central B est une séquence poly(éthylène-butylène) comportant des groupes hydrophiles greffés, ledit copolymère amphiphile ABA pouvant être représenté schématiquement par la structure suivante : 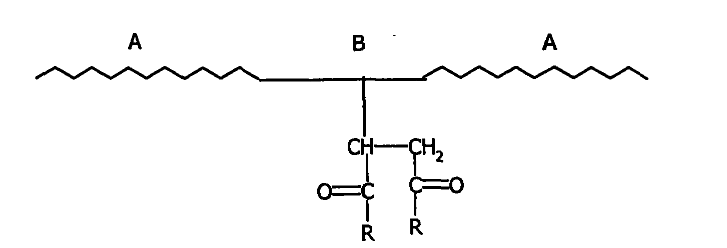
dans laquelle R représente un groupe CH₃-O-(CH₂-CH₂-O)ₙ de masse molaire moyenne égale à 2000 soit n=45 et qui a une masse molaire moyenne mesurée par chromatographie par perméation de gel de 50 000 daltons.

Il existe de multiples domaines dans lesquels les compositions adhésives thermoplastiques selon l'invention peuvent être utilisées dès que le caractère hydrophile permet d'apporter une amélioration à l'adhésif.

Elles présentent généralement de très nombreux avantages et s'avèrent particulièrement utiles chaque fois qu'il est nécessaire d'appliquer, à des fins médicales, dermatologiques, pharmaceutiques ou cosmétiques, un dispositif sur la peau, la plaie ou les muqueuses par exemple un pansement ou un bandage pour le traitement ou la protection de la peau, de la plaie, des brûlures, de l'ampoule, des lésions dermoépidermiques superficielles, profondes, chroniques ou aiguës, pour réaliser un patch pour la libération topique ou systémique d'actifs ou pour le nettoyage ou le soin de la peau comme par exemple un produit désincrustant ou antirides, pour adhésiver une électrode ou pour fixer des produits d'hygiène destinés à entrer en - contact avec la peau comme par exemple dans les couches culottes, pour la réalisation d'adhésifs pour prothèses mammaires ou en stomie par exemple pour des joints employés en ostomles.

Grâce à leur aptitude à contenir de l'eau ou un actif hydrophile dans un milieu à forte teneur hydrophobe et vice versa, dans le cadre de ces applications divers composés peuvent être incorporés lors de la formulation de la composition adhésive hydrophile. Ces composés peuvent être des adjuvants ou des actifs couramment utilisés dans les domaines dermatologiques, cosmétiques ou pharmacologiques. On peut ainsi par exemple incorporer comme adjuvants des antioxydants, des conservateurs, des parfums, des charges, des absorbeurs d'odeurs, des matières colorantes, des filtres UV, des électrolytes pour conduire le courant, des régulateurs de pH, des bactéricides, des particules magnétisables, des microcapsules ou des microsphères.

Les quantités de ces différents adjuvants sont celles classiquement utilisées dans le domaine considéré et par exemple de 0,01 à 20% en poids par rapport au poids total de la composition. Comme agent actif, on peut incorporer un ou plusieurs actifs choisis parmi la liste suivante : vitamines et leurs dérivés, glycérine, collagène, acide salicylique, huiles essentielles aromatiques, caféine, actifs anti-radicaux libres, hydratants, dépigmentants (tel l'acide Kojique), liporégulateurs, anti-acnéiques, anti-vieillissement, adoucissants, décongestionnants, anti-rides, rafraîchissants, agents kératolitiques et agents accélérateurs de cicatrisation, protecteurs vasculaires, anti-bactériens tel la sulfadiazine argentique, anti-fongiques, anti-perspirants, déodorants, agents conditionneurs de la peau, composés insensibilisants, immunomodulateurs et nourrissants, extraits végétaux comme par exemple le thé vert, l'arnica, l'hamamélis..., oligo-éléments, anesthésiques locaux, anti-inflammatoires, hormones, menthol, rétinoïdes, la DHEA, les extraits d'algues, de champignons, de levure, de bactéries, les protéines hydrolysées, partiellement hydrolysées ou non ou les enzymes. Bien entendu, cette liste n'est pas limitative.

Le ou les actifs peuvent être par exemple présents en une concentration allant de 0,01 à 20%, de préférence de 0,1 à 5%, et mieux de 0,5 à 3% du poids total de la composition.

Ces adjuvants ou ces actifs, selon leur nature, peuvent être introduits dans la phase hydrophobe et lipophile ou dans la phase aqueuse. Bien entendu, l'homme du métier veillera à choisir les éventuels actifs ou adjuvants complémentaires et/ou leur quantité de telle manière que les propriétés avantageuses de cohésion, élasticité et adhésion de la composition adhésive thermoplastique hydrophile selon l'invention ne soient pas ou substantiellement pas altérées par l'adjonction envisagée.

Dans le cadre de la réalisation de produits à des fins médicales, dermatologiques ou cosmétiques comme par exemple des patchs, des pansements, des électrodes la couche adhésive hydrophile qui sert de réservoir d'actifs ou d'adjuvants est en général associée à un support.

Etant donné que la composition adhésive est , on réalise dans ce cas l'enduction de la composition adhésive hydrophile sur un support adéquat au grammage souhaité, selon la technique bien connue de l'homme de l'art désignée sous le nom de procédé holt melt.

Le choix du support est réalisé en fonction des propriétés requises (étanchéité, élasticité), suivant le type de produit et l'application recherchée.

Il peut se présenter sous forme d'un film d'une épaisseur variable de 5 à 150 µm ou d'un non-tissé ou d'une mousse ayant une épaisseur de 10 à 500 µm. Ces supports à base de matériaux synthétiques ou naturels sont ceux généralement utilisés par l'homme de l'art dans le domaine des applications visées ci-dessus.

On peut citer ainsi des mousses en polyéthylène, en polyuréthanne, en PVC, des non-tissés en polypropylène, polyamide, polyester ou des complexes réalisés à base d'un film et d'un non-tissé.

D'une façon pratique, la surface de la composition adhésive hydrophile qui n'est pas liée au support, pourra être recouverte d'une couche ou pellicule de protection pelable avant utilisation du produit. L'ensemble ainsi formé pourra être lui-même emballé dans une protection étanche réalisée par exemple au moyen de complexes polyéthylène-aluminium ou dans des blisters.

Les caractéristiques et applications de l'invention seront mieux comprises à la lecture de la description qui va suivre d'exemples de réalisation.

Pour des raisons de simplicité, on donnera l'exemple de synthèse d'un copolymère amphiphile représentatif qui sera utilisé dans toutes les compositions adhésives données à titre d'exemples par la suite.

Le procédé de synthèse de ce copolymère est décrit dans la préparation I ci-après.

Pour réaliser la synthèse de ce dernier, on utilise un réacteur muni d'un réfrigérant, équipé d'un desséchant, d'un sas relié au vide et à l'azote si la réaction est effectuée sous atmosphère inerte, et d'un Dean Stark pour éliminer l'eau formée par distillation azéotropique.

### PREPARATION I

Sous azote on introduit dans le réacteur 150 ml de toluène et on ajoute 20 g de Kraton G 1901^{®} (copolymère SEBS maléisé) commercialisé par la société SHELL. On chauffe à reflux (environ 110 °C) sous agitation jusqu'à dissolution totale du copolymère SEBS maléisé. On prépare à part une solution de PEGME de masse molaire 2 000 commercialisé par la société Aldrich sous la dénomination poly(éthylèneglycol)méthyléther 2000. On dissout ainsi sous agitation 32,32 g de PEGME 2000 en chauffant à sa température de fusion dans 100 ml de toluène. A la solution de copolymère SEBS maléisé préalablement obtenue, on ajoute toujours sous agitation et à reflux, environ 20 gouttes d'acide sulfurique. On ajoute ensuite toujours sous agitation et à reflux la solution de PEGME 2000 dans le toluène préparée précédemment. On a ainsi dans ce cas 4 fonctions hydroxyles pour chaque fonction anhydride. On agite toujours à reflux le mélange obtenu jusqu'à réalisation complète de la réaction d'estérification, soit ici environ 30 à 40 minutes. On précipite alors à chaud, à environ 90 à 100 °C , la solution dans 1,5 litre d'un mélange eau-éthanol 50/50. Après filtration les solvants résiduels sont éliminés du précipité obtenu par évaporation à l'étuve sous vide à 40-50 °C. Pour purifier le polymère amphiphile obtenu il est nécessaire d'éliminer le PEGME 2000 en excès qui n'a pas réagi lors de la synthèse. Pour cela, on redissout à chaud aux environs de 90-100°C, en agitant, le polymère amphiphile dans 100 à 150 ml de toluène et la solution obtenue est à nouveau précipitée dans 1,5 litre d'un mélange eau-éthanol 50/50. Après filtration, le précipité récupéré est séché sous vide à 40-50°C. On répète cette étape de purification (redissolution-précipitation et séchage sous vide) jusqu'à élimination totale du PEGME 2000. On obtient ainsi un copolymère amphiphile dans lequel R₁ et R₂ représentent un groupe CH₃-O-(CH₂-CH₂-O-)ₙ dans lequel n=45 qui a une masse molaire moyenne mesurée par chromatographie par perméation de gel, avec comme solvant du tétrahydrofuranne à un débit de 1 ml par minute, sur une colonne commercialisée par la société WATERS sous la dénomination STYRAGEL HR4 et avec un détecteur à indice de réfraction, de l'ordre de 50 000 daltons.

### EXEMPLES DE COMPOSITIONS SELON L'INVENTION

On a alors réalisé plusieurs compositions adhésives selon l'invention :

### 1) Compositions adhésives dans lesquelles le tackifiant est une résine

Elles sont réalisées selon le procédé de fabrication suivant :

Le mélange des différents constituants est réalisé dans un réacteur fermé, chauffé à une température qui varie entre 100 et 130°C, selon la nature de l'élastomère thermoplastique, de manière à fondre ce dernier.

L'agitation est effectuée grâce à un mélangeur muni d'une hélice défloculeuse à une vitesse d'environ 500 tours par minute.

Dans un premier temps, on introduit dans le réacteur chauffé entre environ 100 et 130°C, le copolymère amphiphile, l'eau, le plastifiant liquide, (dans les exemples suivants une huile minérale) et tous les autres composés, actifs ou adjuvants, non susceptibles de se dégrader à cette température hormis le produit tackifiant qui est ici une résine tackifiante et l'élastomère thermoplastique, puis on agite le mélange ainsi formé de façon continue jusqu'à obtenir un mélange homogène.

Dans un deuxième temps, on introduit alors la résine tackifiante toujours à la même température et l'on continue l'agitation jusqu'à obtention d'un mélange homogène.

On incorpore alors dans ce mélange l'élastomère thermoplastique et on continue à agiter toujours entre 100 à 130°C jusqu'à l'obtention d'un mélange homogène. Si nécessaire l'élastomère thermoplastique peut être mélangé et fondu avec une petite partie du plastifiant liquide avant incorporation pour faciliter son homogénéisation avec le mélange précédent.

Si nécessaire, on laisse refroidir à une température inférieure à 100°C et on incorpore alors tout composé susceptible de se dégrader à une température supérieure comme par exemple des adjuvants, des actifs liposolubles ou hydrosolubles tel un extrait sec d' Hamamélis dans l'exemple 2 et on agite jusqu'à obtention d'un mélange homogène.

Les constituants utilisés pour la réalisation des compositions adhésives hydrophiles décrites ci-après dans les exemples 1 à 4 sont les suivants :-
PREPARATION I copolymère amphiphile
- KRATON D 1161^{®} SIS commercialisé par la société SHELL
- KRATON G 1651^{®} SEBS commercialisé par la société SHELL
- ONDINA 15^{®} huile minérale commercialisée par la société SHELL
- ESCOREZ 5300^{®} résine tackifiante commercialisée par la société EXXON CHEMICAL
- EAU
- METHYLPARABEN conservateur hydrophobe
- PROPYLPARABEN conservateur hydrophile
- EXTRAIT D'HAMAMELIS actif hydrophile commercialisé par la société SEPPIC

Les quantités des différents constituants de ces compositions adhésives hydrophiles exprimées en pourcentage en poids par rapport au poids total de la composition sont rassemblées dans le tableau I.

**TABLEAU I**

| | EX. 1 | EX.2 | EX. 3 | EX.4 |
|---|---|---|---|---|
| PREPARATION I | 2,7 | 4 | 2 | 2 |
| KRATON G 1651^{®} | 2,7 | | | |
| KRATON D 1161^{®} | | 5 | 7 | 4 |
| ONDINA 15^{®} | 32,1 | 46 | 43 | 35 |
| ESCOREZ 5300^{®} | 15,7 | 6 | 12 | 7 |
| EAU | 46,8 | 38,6 | 36 | 52 |
| METHYLPARABEN | | 0,2 | | |
| PROPYLPARABEN | | 0,2 | | |
| EXTRAIT D'HAMAMELIS | | 0,4 | | |

### 2) Composition adhésive dans lesquelles le tackifiant est un polymère hydrosoluble.

### Exemple 5 :

Dans un réacteur fermé, double enveloppe, chauffé à une température qui varie entre 90 et 100°C, on incorpore successivement, sous agitation grâce à un mélangeur muni d'une hélice défloculeuse à une vitesse comprise entre environ 500 à 800 tours par minute, 1 g de copolymère amphiphile obtenu selon la préparation I, 29,4 g d'eau, 7,36g de polymère de polyvinylpyrrolidone commercialisé par la société BASF sous la dénomination Kollidon^{®} 30, 56,8 g de plastifiant (mélange liquide d'hydrocarbures saturés) commercialisé par la société TOTAL sous la dénomination GEMSEAL^{®} 60, 0,2 g de conservateur hydrophobe (Méthylparaben) et 0,2 g de conservateur hydrophile (Propylparaben).

On poursuit cette agitation toujours à une température comprise entre 90 et 100°C jusqu'à obtention d'un mélange homogène.

On introduit alors, toujours à une température comprise entre 90 et 100°C et sous agitation, 5 g de VECTOR^{®} DPX-565, mélange de copolymère triblocs (styrène-isoprène-styrène) et de copolymère diblocs (styrène-isoprène) commercialisé par la société EXXON MOBIL Chemical. On poursuit l'agitation toujours à la même température jusqu'à obtention d'un mélange homogène.

Ce mélange est la composition adhésive hydrophile qui est prête à l'emploi.

### 3) Composition adhésive dans laquelle le tackifiant est un polybutène de bas poids moléculaire.

### Exemple 6 :

Dans un réacteur fermé double enveloppe, équipé d'un mélangeur muni d'une hélice défloculeuse, chauffé à une température qui varie entre 90 et 100°C, on introduit successivement 55,6 g de GEMSEAL^{®} 60 (plastifiant liquide), 10 g de polybutène de bas poids moléculaire commercialisé par la société BP Chemical sous la dénomination NAPVIS^{®} 10.

On introduit ensuite toujours à une température comprise entre 90 et 100°C, en agitant à une vitesse de 800 tours par minute, 1 g de copolymère amphiphile obtenu selon la préparation I, 23 g d'eau, 0,2 g de conservateur hydrophobe (Méthylparaben) et 0,2 g de conservateur hydrophile (Propylparaben) jusqu'à obtention d'un mélange homogène.

On incorpore alors 10 g de VECTOR^{®} DPX-565, toujours à la même température, et on agite toujours à 800 tours par minute jusqu'à obtention d'un mélange homogène qui constitue la composition adhésive hydrophile.

### 4) Composition adhésive hydrophile dans laquelle on a incorporé un tackifiant dans la phase hydrophile et un tackifiant dans la phase hydrophobe

### Exemple 7 :

Dans un réacteur fermé double enveloppe, équipé d'un mélangeur muni d'une hélice défloculeuse, chauffé à une température comprise entre 90 et 100°C, on introduit successivement sous agitation à une vitesse d'environ 800 tours par minute 55,6 g de GEMSEAL^{®} 60 et 10 g de NAPVIS^{®} 10 jusqu'à obtention d'un mélange homogène. On introduit ensuite toujours à une température comprise entre 90 et 100°C et en agitant à 800 tours par minute 1 g de copolymère amphiphile obtenu selon la préparation I, 18,44 g d'eau, 4,56 g de Kollidon^{®} 30, 0,2 g de Méthylparaben et 0,2 g de Propylparaben. On continue l'agitation jusqu'à obtention d'un mélange homogène.

On introduit alors 10 g de VECTOR^{®} DPX-565 et on poursuit l'agitation à la même vitesse, toujours à la même température, jusqu'à obtention d'un mélange homogène qui constitue la composition adhésive hydrophile.

Dans les 3 exemples précédents, on pourra comme précédemment éventuellement mélanger le VECTOR^{®} DPX-565 (élastomère thermoplastique) avec une petite quantité de GEMSEAL^{®} 60 (plastifiant liquide), prise sur la quantité totale incorporée au début des exemples de réalisation, avant son incorporation dans le mélange pour faciliter son homogénéisation avec ce dernier.

Les compositions adhésives hydrophiles sont alors prêtes à être utilisées par exemple incorporées dans un produit, tel qu'un patch.

Dans le cas de l'exemple 2, on a ainsi déposé la composition adhésive qui contient un actif hydrophile et des conservateurs sur un support, ici un non tissé commercialisé par la société KURARAY, pour la réalisation d'un produit patch qui peut être utilisé comme produit de soin, de nettoyage ou de traitement de la peau grâce aux propriétés astringentes, descongestionnantes, calmantes et antibactériennes de l'extrait d'Hamamélis.

L'analyse du tableau I illustre l'originalité des compositions selon la présente invention.

On constate ainsi que. l'on a pu incorporer de l'eau en une quantité allant jusqu'à 52% dans l'exemple 4, dans une composition adhésive .

De même, on a pu incorporer un mélange d'adjuvants hydrophile et hydrophobe, les parabens, et un actif hydrophile l'extrait d'Hamamélis dans l'exemple 2.

Enfin, lorsque le produit à base d'Hamamélis de l'exemple 2 est appliqué sur la peau, on constate qu'il apporte une sensation de fraîcheur très agréable, due à sa teneur relativement élevée en eau (38,6%).

De même, les exemples 5 à 7 illustrent les possibilités de réaliser une composition adhésive hydrophile (le pourcentage d'eau varie de 20 à 30% environ) en jouant sur l'ajout d'un tackifiant dans la phase hydrophile (exemple 5), dans la phase hydrophobe (exemple 6) ou dans les deux (exemple 7).

La présente invention apparaît donc particulièrement avantageuse, dans la mesure où elle permet de disposer pour la première fois dans l'état de la technique de compositions adhésives hydrophiles permettant d'incorporer des quantités prédéterminées d'eau ou de composés hydrophiles à des compositions présentant de bonnes propriétés d'adhésion, de cohésion et d'élasticité et offre de nombreuses possibilités pour régler les propriétés adhésives de ces compositions.

Les compositions ainsi obtenues peuvent être utilisées dans une large gamme d'applications, en particulier pour la réalisation de produits à usage cosmétique, pharmaceutique, dermatologique ou médical, destinés à être mis en contact avec la peau.

## Revendications

1. Composition adhésive hydrophile **caractérisée en ce qu'**elle comprend :
un élastomère thermoplastique, choisi parmi les copolymères séquencés poly(styrène-oléfine-styrène), poly(styrène-oléfine) et leurs mélanges
un produit tackifiant
un plastifiant liquide
de l'eau et
un copolymère amphiphile à blocs du type ABA comportant deux blocs terminaux thermoplastiques A poly(styrène) et un bloc central élastomère B dans lequel le bloc central B est une séquence poly(éthylène-butylène) comportant des groupes hydrophiles greffés, ledit copolymère amphiphile ABA pouvant être représenté schématiquement par la structure suivante : 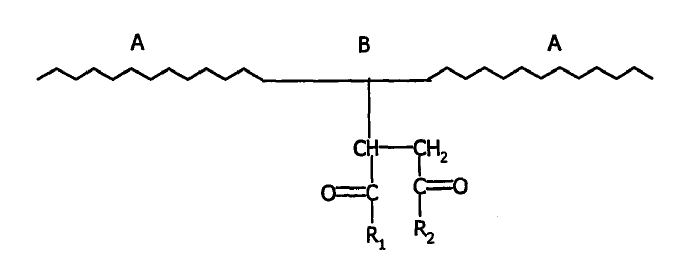
dans laquelle R₁ et R₂ identiques ou différents représentent un groupement hydrophile de masse molaire moyenne inférieure à 10 000, choisi parmi les groupes suivants :
CH₃-O-(CH₂-CH₂-O)ₙ;
HO-(CH₂-CH₂-O)ₙ ; 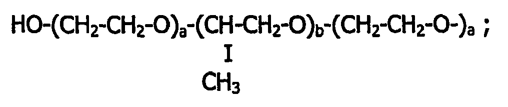
dans lesquels n, a et b représentent un nombre entier.

2. Composition adhésive hydrophile selon la revendication 1 **caractérisée en ce que** le copolymère amphiphile est un copolymère amphiphile à blocs du type ABA comportant deux blocs terminaux thermoplastiques A poly(styrène) et un bloc central élastomère B dans lequel le bloc central B est une séquence poly(éthylène-butylène) comportant des groupes hydrophiles greffés, ledit copolymère amphiphile ABA pouvant être représenté schématiquement par la structure suivante : 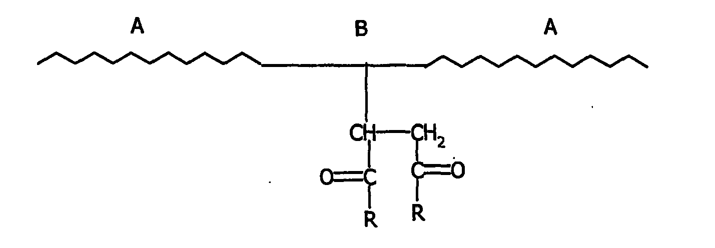 dans laquelle R représente un groupe CH₃-O-(CH₂-CH₂-O)ₙ de masse molaire moyenne comprise de préférence entre 1000 et 8000, de préférence encore égale à 2000 soit n=45.

3. Composition adhésive hydrophile selon la revendication 2 **caractérisée en ce que** le copolymère amphiphile a une masse molaire moyenne mesurée par chromatographie par perméation de gel de l'ordre de 50 000 daltons.

4. Composition adhésive hydrophile selon l'une des revendications précédentes **caractérisée en ce qu'**elle comprend de 0,05 à 20% et de préférence de 0,05 à 5% en poids de copolymère amphiphile par rapport au poids total de la composition.

5. Composition adhésive hydrophile selon l'une des revendications 1 à 4 **caractérisée en ce qu'**elle comprend de 1 à 50% et de préférence de 2 à 30% en poids de produit(s) tackifiant(s) par rapport au poids total de la composition adhésive hydrophile.

6. Composition adhésive hydrophile selon la revendication 5 **caractérisée en ce que** le produit tackifiant est compatible avec la phase hydrophobe de la composition adhésive hydrophile.

7. Composition adhésive hydrophile selon la revendication 6 **caractérisée en ce que** le produit tackifiant est choisi parmi les résines tackifiantes, les polybutènes de bas poids moléculaire ou leurs mélanges.

8. Composition adhésive hydrophile selon la revendication 7 **caractérisée en ce que** le produit tackifiant est une résine tackifiante présente dans une proportion de 5 à 25% en poids par rapport au poids total de la composition adhésive hydrophile.

9. Composition adhésive hydrophile selon la revendication 7 **caractérisée en ce que** le produit tackifiant est un polybutène de bas poids moléculaire présent dans une proportion de 5 à 30% en poids et de préférence de 8 à 15% en poids par rapport au poids total de la composition adhésive hydrophile.

10. Composition adhésive hydrophile selon la revendication 5 **caractérisée en ce que** le produit tackifiant est un produit tackifiant compatible avec la phase aqueuse de la composition adhésive hydrophile et est choisi parmi les polymères solubles dans l'eau, synthétiques non réticulés et notamment parmi les polymères ou copolymères de polyvinylpyrrolidone.

11. Composition adhésive hydrophile selon la revendication 10 **caractérisée en ce qu'**elle comprend de 1 à 30% en poids et de préférence de 2 à 20% en poids de produit tackifiant par rapport au poids total de la composition.

12. Composition adhésive hydrophile selon la revendication 5 **caractérisée en ce qu'**elle comprend de 8 à 25% et de préférence 8 à 15% en poids par rapport au poids total de la composition d'un ensemble de produits tackifiants constitué d'un produit tackifiant compatible avec la phase huileuse et d'un produit tackifiant compatible avec la phase aqueuse et en particulier 2 à 5% en poids par rapport au poids total de la composition de produit tackifiant compatible avec la phase aqueuse et 8 à 12% en poids de produit tackifiant compatible avec la phase huileuse.

13. Composition adhésive hydrophile selon l'une des revendications précédentes **caractérisée en ce que** le plastifiant liquide est une huile plastifiante ou un mélange liquide d'hydrocarbures saturés compatible avec la séquence centrale oléfine de l'élastomère thermoplastique et de préférence une huile plastifiante minérale et en particulier une huile de paraffine.

14. Composition adhésive hydrophile selon la revendication 13 **caractérisée en ce qu'**elle comprend de 20 à 90% en poids et de préférence de 30 à 75% en poids de plastifiant liquide par rapport au poids total de la composition.

15. Composition adhésive hydrophile selon l'une des revendications précédentes **caractérisée en ce que** l'élastomère thermoplastique est un mélange de copolymère poly(styrène-oléfine-styrène) et de copolymère poly(styrène-oléfine)

16. Composition adhésive hydrophile selon l'une des revendications précédentes **caractérisée en ce que** la séquence oléfine est choisie parmi les séquences isoprène, butadiène, éthylène-butylène ou éthylène-propylène et de préférence est une séquence isoprène ou éthylène-butylène.

17. Composition adhésive hydrophile selon l'une des revendications précédentes **caractérisée en ce que** l'élastomère thermoplastique est présent en une concentration de 2 à 20% et en particulier de 5 à 15% en poids par rapport au poids total de la composition et de préférence a une masse molaire moyenne supérieure à celle du copolymère amphiphile et de préférence supérieure ou égale à 100 000 daltons.

18. Composition adhésive hydrophile selon l'une des revendications précédentes **caractérisée en ce qu'**elle comprend de 1 à 50% en poids d'eau et de préférence de 10 à 45% par rapport au poids total de la composition.

19. Composition adhésive hydrophile notamment utilisable sur la peau, la plaie ou les muqueuses **caractérisée en ce qu'**elle comprend :
a. 2 à 20 parties en poids d'un élastomère thermoplastique de masse molaire moyenne supérieure ou égale à 100 000 daltons
b. 30 à 75 parties en poids de plastifiant liquide
c. 2 à 30 parties en poids de produit tackifiant
d. 1 à 45 parties en poids d'eau et
e. 0,05 à 5 parties en poids d'un copolymère amphiphile à blocs du type ABA comportant deux blocs terminaux thermoplastiques A poly(styrène) et un bloc central élastomère B dans lequel le bloc central B est une séquence poly(éthylène-butylène) comportant des groupes hydrophiles greffés, ledit copolymère amphiphile ABA pouvant être représenté schématiquement par la structure suivante : 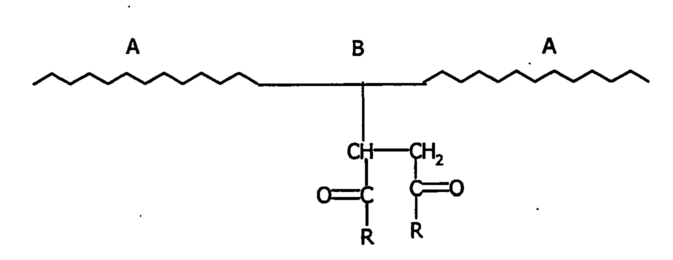
dans laquelle R représente un groupe CH₃-O-(CH₂-CH₂-O)ₙ de masse molaire moyenne égale à 2000 soit n=45 et qui a une masse molaire moyenne mesurée par chromatographie par perméation de gel de 50 000 daltons.

20. Composition adhésive hydrophile selon la revendication 19 **caractérisée en qu'**elle comprend :
a. 2 à 20 parties en poids d'un élastomère thermoplastique de masse molaire moyenne supérieure ou égale à 100 000 daltons
b. 30 à 75 parties en poids de plastifiant liquide
c. 5 à 25 parties en poids de résine tackifiante
d. 10 à 40 parties en poids d'eau et
e. 0,05 à 5 parties en poids d'un copolymère amphiphile à blocs du type ABA comportant deux blocs terminaux thermoplastiques A poly(styrène) et un bloc central élastomère B dans lequel le bloc central B est une séquence poly(éthylène-butylène) comportant des groupes hydrophiles greffés, ledit copolymère amphiphile ABA pouvant être représenté schématiquement par la structure suivante : 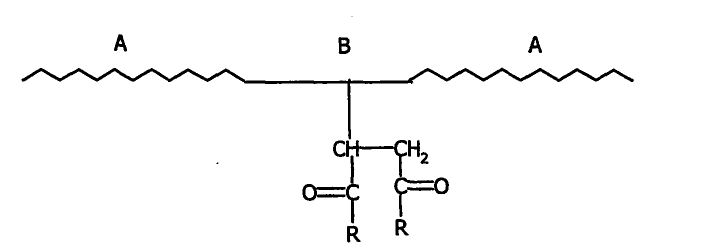
dans laquelle R représente un groupe CH₃-O-(CH₂-CH₂-O)ₙ de masse molaire moyenne égale à 2000 soit n=45 et qui a une masse molaire moyenne mesurée par chromatographie par perméation de gel de 50 000 daltons.

21. Composition adhésive hydrophile selon la revendication 19 **caractérisée en qu'**elle comprend :
a. 2 à 20 parties en poids d'un élastomère thermoplastique de masse molaire moyenne supérieure ou égale à 100 000 daltons
b. 30 à 75 parties en poids de plastifiant liquide
c. 8 à 15 parties en poids de polybutène de bas poids moléculaire
d. 10 à 40 parties en poids d'eau et
e. 0,05 à 5 parties en poids d'un copolymère amphiphile à blocs du type ABA comportant deux blocs terminaux thermoplastiques A poly(styrène) et un bloc central élastomère B dans lequel le bloc central B est une séquence poly(éthylène-butylène) comportant des groupes hydrophiles greffés, ledit copolymère amphiphile ABA pouvant être représenté schématiquement par la structure suivante : 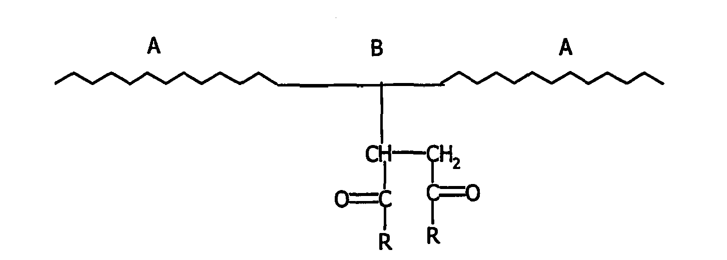
dans laquelle R représente un groupe CH₃-O-(CH₂-CH₂-O)ₙ de masse molaire moyenne égale à 2000 soit n=45 et qui a une masse molaire moyenne mesurée par chromatographie par perméation de gel de 50 000 daltons.

22. Composition adhésive hydrophile selon la revendication 19 **caractérisée en qu'**elle comprend :
a. 2 à 20 parties en poids d'un élastomère thermoplastique de masse molaire moyenne supérieure ou égale à 100 000 daltons
b. 30 à 75 parties en poids de plastifiant liquide
c. 2 à 20 parties en poids de polymère hydrosoluble
d. 10 à 40 parties en poids d'eau et
e. 0,05 à 5 parties en poids d'un copolymère amphiphile à blocs du type ABA comportant deux blocs terminaux thermoplastiques A poly(styrène) et un bloc central élastomère B dans lequel le bloc central B est une séquence poly(éthylène-butylène) comportant des groupes hydrophiles greffés, ledit copolymère amphiphile ABA pouvant être représenté schématiquement par la structure suivante : 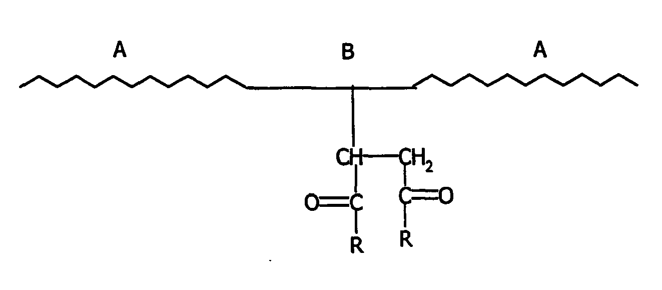
dans laquelle R représente un groupe CH₃-O-(CH₂-CH₂-O)ₙ de masse molaire moyenne égale à 2000 soit n=45 et qui a une masse molaire moyenne mesurée par chromatographie par perméation de gel de 50 000 daltons.

23. Composition adhésive hydrophile selon la revendication 19 **caractérisée en qu'**elle comprend :
a. 2 à 20 parties en poids d'un élastomère thermoplastique de masse molaire moyenne supérieure ou égale à 100 000 daltons
b. 30 à 75 parties en poids de plastifiant liquide
c. 8 à 15 parties en poids d'un ensemble de produits tackifiants constitué d'un polybutène de bas poids moléculaire et d'un polymère hydrosoluble et tout particulièrement de 10 parties en poids de polybutène de bas poids moléculaire et de 2 à 5 parties en poids de polymère de vinylpyrrolidone.
d. 10 à 40 parties en poids d'eau et
e. 0,05 à 5 parties en poids d'un copolymère amphiphile à blocs du type ABA comportant deux blocs terminaux thermoplastiques A poly(styrène) et un bloc central élastomère B dans lequel le bloc central B est une séquence poly(éthylène-butylène) comportant des groupes hydrophiles greffés, ledit copolymère amphiphile ABA pouvant être représenté schématiquement par la structure suivante : 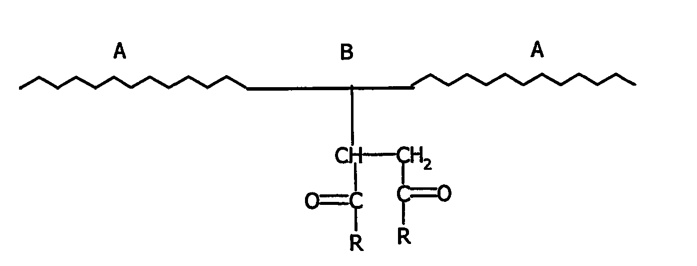
dans laquelle R représente un groupe CH₃-O-(CH₂-CH₂-O)ₙ de masse molaire moyenne égale à 2000 soit n=45 et qui a une masse molaire moyenne mesurée par chromatographie par perméation de gel de 50 000 daltons.

24. Utilisation d'une composition adhésive hydrophile selon l'une des revendications 1 à 23, pour la fabrication de produits destinés à des fins médicales, dermatologiques, cosmétologiques ou pharmaceutiques.

25. Utilisation d'une composition adhésive hydrophile selon l'une des revendications 1 à 23, pour la fabrication d'un produit à appliquer sur la peau, la plaie ou les muqueuses

26. Utilisation d'une composition adhésive hydrophile selon l'une des revendications 24 ou 25 **caractérisée en ce que** le produit est un pansement pour le traitement ou la protection de la plaie, de l'ampoule, des brûlures, ou des lésions dermo-épidermiques superficielles, un patch pour la délivrance d'actifs par voie topique ou systémique, un produit de soin, de nettoyage ou de protection de la peau ou des muqueuses, une électrode, ou un produit pour stomie.

## Claims

1. A hydrophilic adhesive composition, **characterised in that** it comprises :
a thermoplastic elastomer, which is selected from the block copolymers poly(styrene-olefin-styrene), poly(styrene-olefin) and
their mixtures,
a tackifying product,
a liquid plasticizer,
water, and
an amphiphilic ABA type block copolymer comprising two terminal thermoplastic poly(styrene) blocks A and one central elastomeric block B in which the central block B is a poly(ethylene-butylene) sequence comprising grafted hydrophilic groups, it being possible for said amphiphilic copolymer ABA to be represented schematically by the following structure : 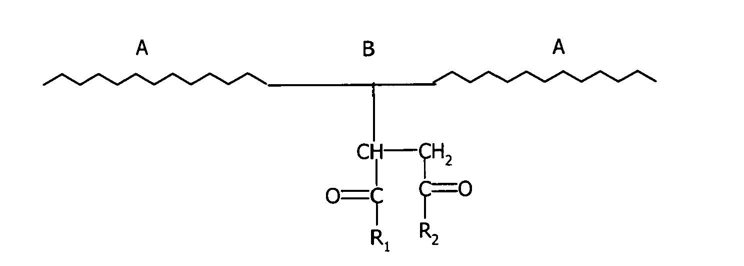
in which R₁ and R₂, which are identical or different, represent a hydrophilic group of average molar mass less than 10,000, selected from the following groups :
CH₃-O-(CH₂-CH₂-O)ₙ ;
HO-(CH₂-CH₂-O)ₙ ; 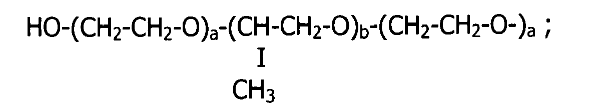
in which n, a and b represent an integer.

2. The hydrophilic adhesive composition according to claim 1, **characterised in that** the amphiphilic copolymer is an amphiphilic ABA type block copolymer comprising two terminal thermoplastic poly(styrene) blocks A and one central elastomeric block B in which the central block B is a poly(ethylene-butylene) sequence comprising grafted hydrophilic groups, it being possible for said amphiphilic copolymer ABA to be represented schematically by the following structure : 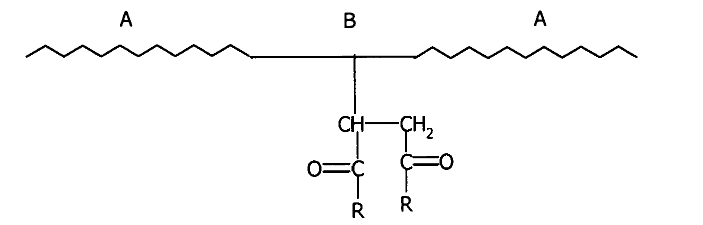 in which R represents a CH₃-O-(CH₂-CH₂-O)ₙ group of average molar mass preferably of between 1,000 and 8,000, more preferably equal to 2,000, i.e. n=45.

3. The hydrophilic adhesive composition according to claim 2, **characterised in that** the amphiphilic copolymer has an average molar mass measured by gel permeation chromatography of the order of 50,000 daltons.

4. The hydrophilic adhesive composition according to one of the preceding claims, **characterised in that** it comprises 0.05 to 20%, preferably 0.05 to 5%, by weight of amphiphilic copolymer with respect to the total weight of the composition.

5. The hydrophilic adhesive composition according to one of claims 1 to 4, **characterised in that** it comprises 1 to 50%, preferably 2 to 30%, by weight of tackifying product(s) with respect to the total weight of the hydrophilic adhesive composition.

6. The hydrophilic adhesive composition according to claim 5, **characterised in that** the tackifying product is compatible with the hydrophobic phase of the hydrophilic adhesive composition.

7. The hydrophilic adhesive composition according to claim 6, **characterised in that** the tackifying product is selected from tackifying resins, low molecular weight polybutenes or their mixtures.

8. The hydrophilic adhesive composition according to claim 7, **characterised in that** the tackifying product is a tackifying resin which is present in a proportion of 5 to 25% by weight with respect to the total weight of the hydrophilic adhesive composition.

9. The hydrophilic adhesive composition according to claim 7, **characterised in that** the tackifying product is a low molecular weight polybutene present in a proportion of 5 to 30% by weight, preferably 8 to 15% by weight, with respect to the total weight of the hydrophilic adhesive composition.

10. The hydrophilic adhesive composition according to claim 5, **characterised in that** the tackifying product is a tackifying product which is compatible with the aqueous phase of the hydrophilic adhesive composition and is selected from non-cross-linked, synthetic watersoluble polymers, and notably from polyvinylpyrrolidone polymers or polyvinylpyrrolidone copolymers.

11. The hydrophilic adhesive composition according to claim 10, **characterised in that** it comprises 1 to 30% by weight, preferably 2 to 20% by weight of tackifying product with respect to the total weight of the composition.

12. The hydrophilic adhesive composition according to claim 5, **characterised in that** it comprises 8 to 25%, preferably 8 to 15%, by weight with respect to the total weight of the composition of a group of tackifying products which is constituted of a tackifying product which is compatible with the oily phase and of a tackifying product which is compatible with the aqueous phase, and particularly 2 to 5% by weight with respect to the total weight of the composition of tackifying product compatible with the aqueous phase and 8 to 12% by weight of tackifying product compatible with the oily phase.

13. The hydrophilic adhesive composition according to one of the preceding claims, **characterised in that** the liquid plasticizer is a plasticizing oil or a liquid mixture of saturated hydrocarbons which is compatible with the central olefinic sequence of the thermoplastic elastomer, preferably a mineral plasticizing oil, particularly a liquid paraffin.

14. The hydrophilic adhesive composition according to claim 13, **characterised in that** it comprises 20 to 90% by weight, preferably 30 to 75% by weight, of liquid plasticizer with respect to the total weight of the composition.

15. The hydrophilic adhesive composition according to one of the preceding claims, **characterised in that** the thermoplastic elastomer is a mixture of poly(styrene-olefin-styrene) copolymer and of poly(styrene-olefin) copolymer.

16. The hydrophilic adhesive composition according to one of the preceding claims, **characterised in that** the olefinic sequence is selected from the sequences : isoprene, butadiene, ethylene-butylene or ethylenepropylene and is preferably an isoprene or ethylene-butylene sequence.

17. The hydrophilic adhesive composition according to one of the preceding claims, **characterised in that** the thermoplastic elastomer is present at a concentration of 2 to 20%, particularly 5 to 15%, by weight with respect to the total weight of the composition and preferably has an average molar mass greater than that of the amphiphilic copolymer, preferably greater than or equal to 100,000 daltons.

18. The hydrophilic adhesive composition according to one of the preceding claims, **characterised in that** it comprises 1 to 50% by weight of water, preferably 10 to 45%, with respect to the total weight of the composition.

19. A hydrophilic adhesive composition usable notably on the skin, a wound or the mucous membranes, **characterised in that** it comprises :
a. 2 to 20 parts by weight of a thermoplastic elastomer of average molar mass greater than or equal to 100,000 daltons,
b. 30 to 75 parts by weight of liquid plasticizer,
c. 2 to 30 parts by weight of tackifying product,
d. 1 to 45 parts by weight of water, and
e. 0.05 to 5 parts by weight of an amphiphilic ABA type block copolymer comprising two terminal thermoplastic poly(styrene) blocks A and one central elastomeric block B in which the central block B is a poly(ethylene-butylene) sequence comprising grafted hydrophilic groups, it being possible for said amphiphilic copolymer ABA to be represented schematically by the following structure : 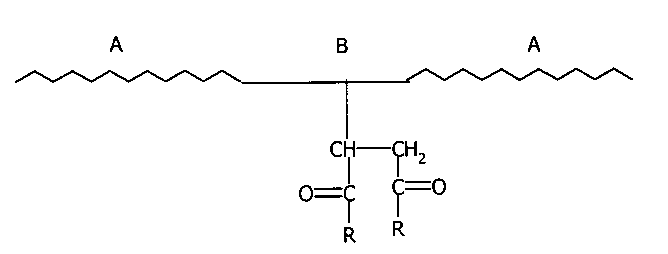
in which R represents a CH₃-O-(CH₂-CH₂-O)ₙ group of average molar mass equal to 2,000, i.e. n=45, and which has an average molar mass measured by gel permeation chromatography of 50,000 daltons.

20. The hydrophilic adhesive composition according to la claim 19, **characterised in that** it comprises :
a. 2 to 20 parts by weight of a thermoplastic elastomer of average molar mass greater than or equal to 100,000 daltons,
b. 30 to 75 parts by weight of liquid plasticizer,
c. 5 to 25 parts by weight of tackifying resin,
d. 10 to 40 parts by weight of water, and
e. 0.05 to 5 parts by weight of an amphiphilic ABA type block copolymer comprising two terminal thermoplastic poly(styrene) blocks A and one central elastomeric block B in which the central block B is a poly(ethylene-butylene) sequence comprising grafted hydrophilic groups, it being possible for said amphiphilic copolymer ABA to be represented schematically by the following structure : 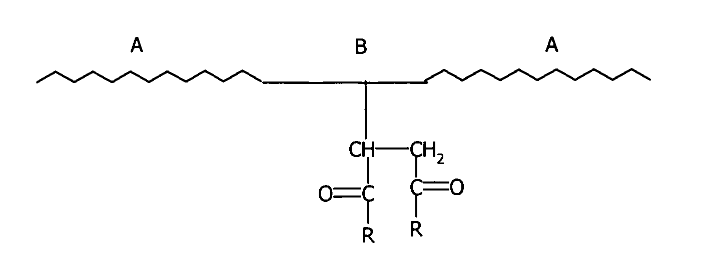
in which R represents a CH₃-O-(CH₂-CH₂-O)ₙ group of average molar mass equal to 2,000, i.e. n=45, and which has an average molar mass measured by gel permeation chromatography of 50,000 daltons.

21. The hydrophilic adhesive composition according to claim 19, **characterised in that** it comprises :
a. 2 to 20 parts by weight of a thermoplastic elastomer of average molar mass greater than or equal to 100,000 daltons,
b. 30 to 75 parts by weight of liquid plasticizer,
c. 8 to 15 parts by weight of low molecular weight polybutene,
d. 10 to 40 parts by weight of water, and
e. 0.05 to 5 parts by weight of an amphiphilic ABA type block copolymer comprising two terminal thermoplastic poly(styrene) blocks A and one central elastomeric block B in which the central block B is a poly(ethylene-butylene) sequence comprising grafted hydrophilic groups, it being possible for said amphiphilic copolymer ABA to be represented schematically by the following structure : 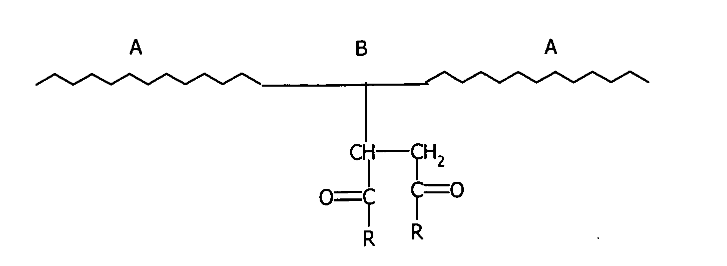
in which R represents a CH₃-O-(CH₂-CH₂-O)ₙ group of average molar mass equal to 2,000, i.e. n=45, and which has an average molar mass measured by gel permeation chromatography of 50,000 daltons.

22. The hydrophilic adhesive composition according to claim 19, **characterised in that** it comprises :
a. 2 to 20 parts by weight of a thermoplastic elastomer of average molar mass greater than or equal to 100,000 daltons,
b. 30 to 75 parts by weight of liquid plasticizer,
c. 2 to 20 parts by weight of hydrosoluble polymer,
d. 10 to 40 parts by weight of water, and
e. 0.05 to 5 parts by weight of an amphiphilic ABA type block copolymer comprising two terminal thermoplastic poly(styrene) blocks A and one central elastomeric block B in which the central block B is a poly(ethylene-butylene) sequence comprising grafted hydrophilic groups, it being possible for said amphiphilic copolymer ABA to be represented schematically by the following structure : 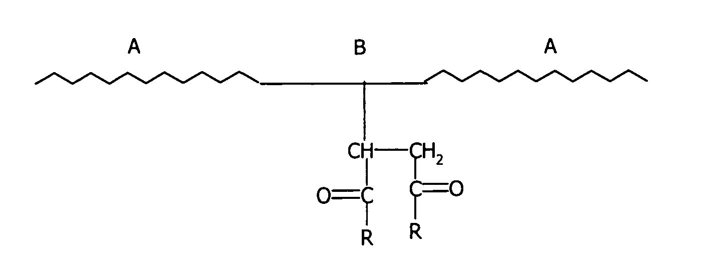
in which R represents a CH₃-O-(CH₂-CH₂-O)ₙ group of average molar mass equal to 2,000, i.e. n=45, and which has an average molar mass measured by gel permeation chromatography of 50,000 daltons.

23. The hydrophilic adhesive composition according to claim 19, **characterised in that** it comprises :
a. 2 to 20 parts by weight of a thermoplastic elastomer of average molar mass greater than or equal to 100,000 daltons,
b. 30 to 75 parts by weight of liquid plasticizer,
c. 8 to 15 parts by weight of a group of tackifying products which is constituted of a low molecular weight polybutene and of a hydrosoluble polymer and more particularly 10 parts by weight of low molecular weight polybutene and 2 to 5 parts by weight of vinylpyrrolidone polymer,
d. 10 to 40 parts by weight of water, and
e. 0.05 to 5 parts by weight of an amphiphilic ABA type block copolymer comprising two terminal thermoplastic poly(styrene) blocks A and one central elastomeric block B in which the central block B is a poly(ethylene-butylene) sequence comprising grafted hydrophilic groups, it being possible for said amphiphilic copolymer ABA to be represented schematically by the following structure : 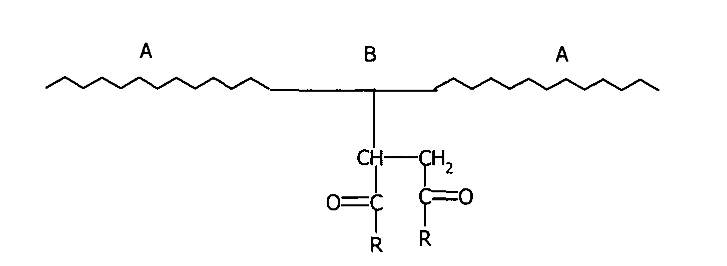
in which R represents a CH₃-O-(CH₂-CH₂-O)ₙ group of average molar mass equal to 2,000, i.e. n=45, and which has an average molar mass measured by gel permeation chromatography of 50,000 daltons.

24. Use of a hydrophilic adhesive composition according to one of claims 1 to 23 for the manufacture of products intended for medical, dermatological, cosmetological or pharmaceutical purposes.

25. Use of a hydrophilic adhesive composition according to one of claims 1 to 23 for the manufacture of a product to be applied on the skin, a wound or the mucous membranes.

26. Use of a hydrophilic adhesive composition according to one of claims 24 or 25, **characterised in that** the product is a dressing for treating or protecting a wound, a blister, burns, or superficial dermo-epidermic lesions, a patch for delivering actives via the topical or systemic route, a product for the care, the cleansing or the protection of the skin or the mucous membranes, an electrode, or a product for stoma.

## Patentansprüche

1. Hydrophile Klebstoffzusammensetzung, **dadurch gekennzeichnet, daß** sie umfaßt:
- ein thermoplastisches Elastomer, gewählt unter den sequentiellen Copolymeren Poly-(Styrol-Olefin-Styrol), Poly-(Styrol-Olefin) und deren Gemischen,
- ein klebrigmachendes Produkt,
- einen Flüssigweichmacher,
- Wasser und
- ein amphiphiles Blockcopolymer vom Typ ABA, umfassend zwei thermoplastische terminale Poly-(Styrol)-Blöcke A und einen zentralen Elastomerblock B, bei welchem der zentrale Block B eine Sequenz Poly-(Ethylen-Butylen) ist, umfassend gepfropfte hydrophile Gruppen, wobei das amphiphile Copolymer ABA schematisch dargestellt werden kann durch die folgende Struktur: 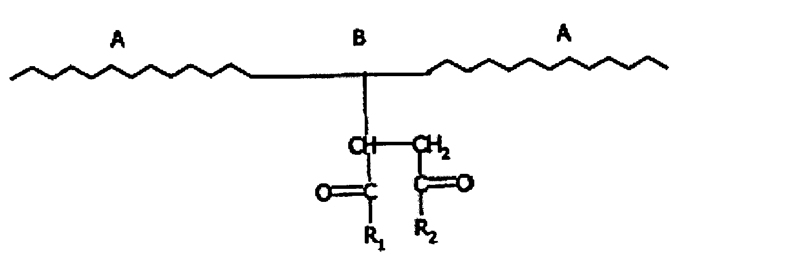
in welcher R₁ und R₂ identisch oder verschieden eine hydrophile Gruppe mit einer mittleren molaren Masse unter 10.000 darstellen, gewählt unter den folgenden Gruppen:
CH₃-O-(CH₂-CH₂-O)ₙ;
HO-(CH₂-CH₂-O)n; 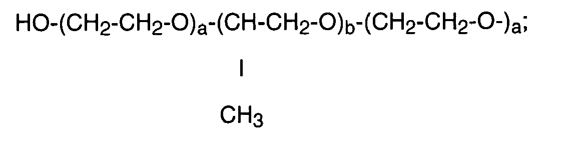
in denen n, a und b eine ganze Zahl darstellen.

2. Hydrophile Klebstoffzusammensetzung nach Anspruch 1, **dadurch gekennzeichnet, daß** das amphiphile Copolymer ein amphiphiles Copolymer mit Blöcken vom Typ ABA ist, umfassend zwei thermoplastische terminale Poly-(Styrol)-Blöcke A und einen zentralen Elastomerblock B, bei welchem der zentrale Block B eine Sequenz Poly-(Ethylen-Butylen) ist, umfassend gepfropfte hydrophile Gruppen, wobei das amphiphile Copolymer ABA schematisch durch die folgende Struktur dargestellt werden kann: 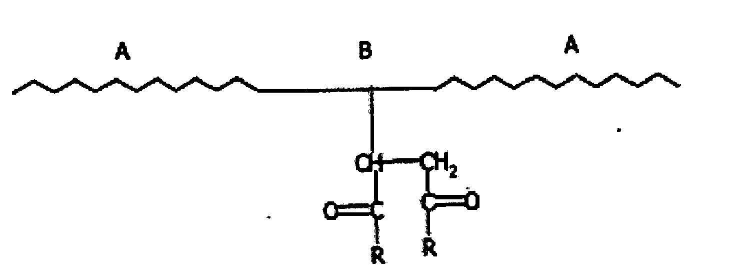 in welcher R eine Gruppe CH₃-O-(CH₂-CH₂-O)ₙ mit mittlerer molarer Masse zwischen vorzugsweise 1.000 und 8.000, noch bevorzugter gleich 2.000, das heißt n = 45, darstellt.

3. Hydrophile Klebstoffzusammensetzung nach Anspruch 2, **dadurch gekennzeichnet, daß** das amphiphile Copolymer eine mittlere molare Masse , gemessen durch Gelpermeationschromatographie in der Größenordnung von 50.000 Dalton hat.

4. Hydrophile Klebstoffzusammensetzung nach einem der vorhergehenden Ansprüche, **dadurch gekennzeichnet, daß** sie 0,05 bis 20 Gewichtsprozent und vorzugsweise 0,05 bis 5 Gewichtsprozent amphiphiles Polymer im Verhältnis zum Gesamtgewicht der Zusammensetzung umfaßt.

5. Hydrophile Klebstoffzusammensetzung nach einem der Ansprüche 1 bis 4, **dadurch gekennzeichnet, daß** sie 1 bis 50 Gewichtsprozent und vorzugsweise 2 bis 30 Gewichtsprozent klebrigmachendes (klebrigmachende) Produkt(e) im Verhältnis zum Gesamtgewicht der hydrophilen Klebstoffzusammensetzung umfaßt.

6. Hydrophile Klebstoffzusammensetzung nach Anspruch 5, **dadurch gekennzeichnet, daß** das klebrigmachende Produkt kompatibel mit der hydrophoben Phase der hydrophilen Klebstoffzusammensetzung ist.

7. Hydrophile Klebstoffzusammensetzung nach Anspruch 6, **dadurch gekennzeichnet, daß** das klebrigmachende Produkt unter den Klebharzen, den Polybutenen niedrigen Molekulargewichts und deren Gemischen gewählt ist.

8. Hydrophile Klebstoffzusammensetzung nach Anspruch 7, **dadurch gekennzeichnet, daß** das klebrigmachende Produkt ein Polybuten niedrigen Molekulargewichts ist, das in einem Anteil von 5 bis 25 Gewichtsprozent im Verhältnis zum Gesamtgewicht der hydrophilen Klebstoffzusammensetzung vorliegt.

9. Hydrophile Klebstoffzusammensetzung nach Anspruch 7, **dadurch gekennzeichnet, daß** das klebrigmachende Produkt ein Klebharz ist, das in einem Anteil von 5 bis 30 Gewichtsprozent und vorzugsweise 8 bis 15 Gewichtsprozent im Verhältnis zum Gesamtgewicht der hydrophilen Klebstoffzusammensetzung vorliegt.

10. Hydrophile Klebstoffzusammensetzung nach Anspruch 5, **dadurch gekennzeichnet, daß** das klebrigmachende Produkt ein klebrigmachendes Produkt ist, das kompatibel mit der wäßrigen Phase der hydrophilen Klebstoffzusammensetzung und gewählt ist unter den wasserlöslichen, synthetischen nicht vernetzten Polymeren und insbesondere unter den Polymeren oder Copolymeren von Polyvinylpyrrolidon.

11. Hydrophile Klebstoffzusammensetzung nach Anspruch 10, **dadurch gekennzeichnet, daß** sie 1 bis 30 Gewichtsprozent und vorzugsweise 2 bis 20 Gewichtsprozent eines klebrigmachenden Produktes im Verhältnis zum Gesamtgewicht der Zusammensetzung umfaßt.

12. Hydrophile Klebstoffzusammensetzung nach Anspruch 5, **dadurch gekennzeichnet, daß** sie im Verhältnis zum Gesamtgewicht der Zusammensetzung 8 bis 25 Gewichtsprozent und vorzugsweise 8 bis 15 Gewichtsprozent von einer Gesamtheit von klebrigmachenden Produkten umfaßt, bestehend aus einem klebrigmachenden Produkt, das kompatibel ist mit der öligen Phase und einem klebrigmachenden Produkt, das kompatibel ist mit der wäßrigen Phase, und insbesondere 2 bis 5 Gewichtsprozent im Verhältnis zum Gesamtgewicht der Zusammensetzung von klebrigmachendem Produkt, das kompatibel ist mit der wäßrigen Phase, und 8 bis 12 Gewichtsprozent von klebrigmachendem Produkt, das kompatibel ist mit der öligen Phase.

13. Hydrophile Klebstoffzusammensetzung nach einem der vorhergehenden Ansprüche, **dadurch gekennzeichnet, daß** der Flüssigweichmacher ein Weichmacheröl oder ein Flüssiggemisch von gesättigten Kohlenwasserstoffen, das kompatibel mit der zentralen Olefinsequenz des thermoplastischen Elastomers ist und vorzugsweise ein Mineralweichmacheröl und insbesondere ein Paraffinöl ist.

14. Hydrophile Klebstoffzusammensetzung nach Anspruch 13, **dadurch gekennzeichnet, daß** sie 20 bis 90 Gewichtsprozent und vorzugsweise 30 bis 75 Gewichtsprozent eines Flüssigweichmachers im Verhältnis zum Gesamtgewicht der Zusammensetzung umfaßt.

15. Hydrophile Klebstoffzusammensetzung nach einem der vorhergehenden Ansprüche, **dadurch gekennzeichnet, daß** das thermoplastische Elastomer ein Gemisch von Poly-(Styrol-Olefin-Styrol)-Copolymer und Poly-(Styrol-Olefin)-Copolymer ist.

16. Hydrophile Klebstoffzusammensetzung nach einem der vorhergehenden Ansprüche, **dadurch gekennzeichnet, daß** die Olefinsequenz gewählt wird unter den Sequenzen Isopren, Butadien, Ethylen-Butylen oder Ethylen-Propylen und vorzugsweise eine Sequenz Isopren oder Ethylen-Butylen ist.

17. Hydrophile Klebstoffzusammensetzung nach einem der vorhergehenden Ansprüche, **dadurch gekennzeichnet, daß** das thermoplastische Elastomer in einer Konzentration von 2 bis 20 Gewichtsprozent und insbesondere 5 bis 15 Gewichtsprozent im Verhältnis zum Gesamtgewicht der Zusammensetzung vorliegt und vorzugsweise eine mittlere molaren Masse größer als jene des amphiphilen Copolymers und vorzugsweise über oder gleich 100.000 Dalton besitzt.

18. Hydrophile Klebstoffzusammensetzung nach einem der vorhergehenden Ansprüche, **dadurch gekennzeichnet, daß** sie 1 bis 50 Gewichtsprozent Wasser und vorzugsweise 10 bis 45% im Verhältnis zum Gesamtgewicht der Zusammensetzung umfaßt.

19. Hydrophile Klebstoffzusammensetzung, insbesondere verwendbar für die Haut, die Wunde oder die Schleimhäute, **dadurch gekennzeichnet, daß** sie umfaßt:
a. 2 bis 20 Gewichtsteile eines thermoplastischen Elastomers einer mittleren molaren Masse größer oder gleich 100.000 Dalton,
b. 30 bis 75 Gewichtsteile eines Flüssigweichmachers,
c. 2 bis 30 Gewichtsteile eines klebrigmachenden Produkts,
d. 1 bis 45 Gewichtsteile Wasser und
e. 0,05 bis 5 Gewichtsteile eines amphiphilen Blockcopolymers vom Typ ABA, umfassend zwei thermoplastische terminale Poly-(Styrol)-Blöcke A und einen zentralen Elastomerblock B, bei welchem der zentrale Block B eine Sequenz Poly-(Ethylen-Butylen) ist, umfassend gepfropfte hydrophile Gruppen, wobei das amphiphile Copolymer ABA schematisch dargestellt werden kann durch die folgende Struktur: 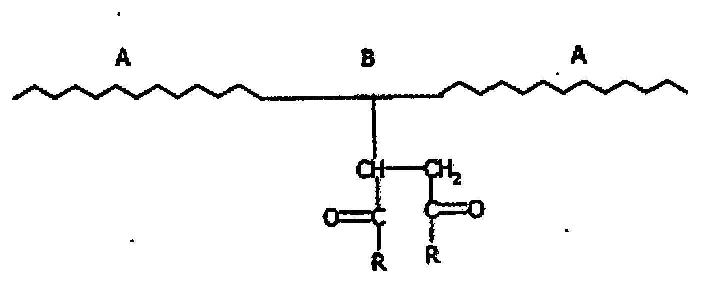
in welcher R eine Gruppe CH₃-O-(CH₂-CH₂-O)ₙ mit mittlerer molarer Masse gleich 2.000, das heißt n = 45, darstellt und dessen durch Gelpermeationschromatographie gemessene mittlere molare Masse 50.000 Dalton ist.

20. Hydrophile Klebstoffzusammensetzung nach Anspruch 19, **dadurch gekennzeichnet, daß** sie umfaßt:
a. 2 bis 20 Gewichtsteile eines thermoplastischen Elastomers einer mittleren molaren Masse größer oder gleich 100.000 Dalton,
b. 30 bis 75 Gewichtsteile eines Flüssigweichmachers,
c. 5 bis 25 Gewichtsteile eines klebrigmachenden Harzes,
d. 10 bis 40 Gewichtsteile Wasser und
e. 0,05 bis 5 Gewichtsteile eines amphiphilen Blockcopolymers vom Typ ABA, umfassend zwei thermoplastische terminale Poly-(Styrol)-Blöcke A und einen zentralen Elastomerblock B, bei welchem der zentrale Block B eine Sequenz Poly-(Ethylen-Butylen) ist, umfassend gepfropfte hydrophile Gruppen, wobei das amphiphile Copolymer ABA schematisch dargestellt werden kann durch die folgende Struktur: 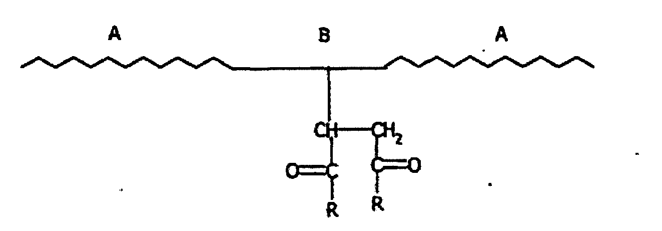
in welcher R eine Gruppe CH₃-O-(CH₂-CH₂-O)ₙ mit mittlerer molarer Masse gleich 2.000, das heißt n = 45, darstellt und das eine durch Gelchromatographie gemessene mittlere molare Masse von 50.000 Dalton hat.

21. Hydrophile Klebstoffzusammensetzung nach Anspruch 19, **dadurch gekennzeichnet, daß** sie umfaßt:
a. 2 bis 20 Gewichtsteile eines thermoplastischen Elastomers einer mittleren molaren Masse größer oder gleich 100.000 Dalton,
b. 30 bis 75 Gewichtsteile eines Flüssigweichmachers,
c. 8 bis 15 Gewichtsteile Polybuten niedrigen Molekulargewichts,
d. 10 bis 40 Gewichtsteile Wasser und
e. 0,05 bis 5 Gewichtsteile eines amphiphilen Blockcopolymers vom Typ ABA, umfassend zwei thermoplastische terminale Poly-(Styrol)-Blöcke A und einen zentralen Elastomerblock B, bei welchem der zentrale Block B eine Sequenz Poly-(Ethylen-Butylen) ist, umfassend gepfropfte hydrophile Gruppen, wobei das amphiphile Copolymer ABA schematisch dargestellt werden kann durch die folgende Struktur: 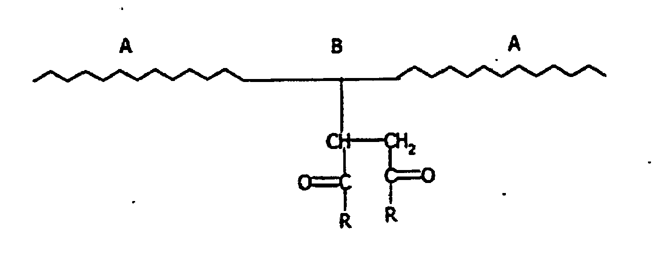
in welcher R eine Gruppe CH₃-O-(CH₂-CH₂-O)ₙ mit mittlerer molarer Masse gleich 2.000, das heißt n = 45, darstellt und das eine durch Gelchromatographie gemessene mittlere molare Masse von 50.000 Dalton hat.

22. Hydrophile Klebstoffzusammensetzung nach Anspruch 19, **dadurch gekennzeichnet, daß** sie umfaßt:
a. 2 bis 20 Gewichtsteile eines thermoplastischen Elastomers einer mittleren molaren Masse größer oder gleich 100.000 Dalton,
b. 30 bis 75 Gewichtsteile eines Flüssigweichmachers,
c. 2 bis 20 Gewichtsteile wasserlösliches Polymer,
d. 10 bis 40 Gewichtsteile Wasser und
e. 0,05 bis 5 Gewichtsteile eines amphiphilen Blockcopolymers vom Typ ABA, umfassend zwei thermoplastische terminale Poly-(Styrol)-Blöcke A und einen zentralen Elastomerblock B, bei welchem der zentrale Block B eine Sequenz Poly-(Ethylen-Butylen) ist, umfassend gepfropfte hydrophile Gruppen, wobei das amphiphile Copolymer ABA schematisch dargestellt werden kann durch die folgende Struktur: 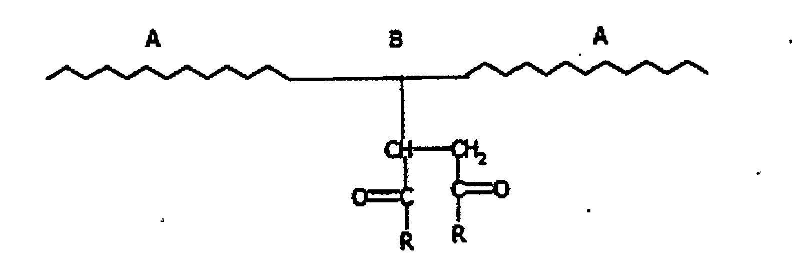
in welcher R eine Gruppe CH₃-O-(CH₂-CH₂-O)ₙ mit mittlerer molarer Masse gleich 2.000, das heißt n = 45, darstellt und das eine durch Gelchromatographie gemessene mittlere molare Masse von 50.000 Dalton hat.

23. Hydrophile Klebstoffzusammensetzung nach Anspruch 19, **dadurch gekennzeichnet, daß** sie umfaßt:
a. 2 bis 20 Gewichtsteile eines thermoplastischen Elastomers einer mittleren molaren Masse größer oder gleich 100.000 Dalton,
b. 30 bis 75 Gewichtsteile eines Flüssigweichmachers,
c. 8 bis 15 Gewichtsteile einer Gesamtheit klebrigmachender Produkte, bestehend aus einem Polybuten niedrigen Molekulargewichts und einem wasserlöslichen Polymer und ganz speziell 10 Gewichtsteilen Polybuten niedrigen Molekulargewichts und 2 bis 5 Gewichtsteilen Vinylpyrrolidon,
d. 10 bis 40 Gewichtsteile Wasser und
e. 0,05 bis 5 Gewichtsteile eines amphiphilen Blockcopolymers vom Typ ABA, umfassend zwei thermoplastische terminale Poly-(Styrol)-Blöcke A und einen zentralen Elastomerblock B, bei welchem der zentrale Block B eine Sequenz Poly-(Ethylen-Butylen) ist, umfassend gepfropfte hydrophile Gruppen, wobei das amphiphile Copolymer ABA schematisch dargestellt werden kann durch die folgende Struktur: 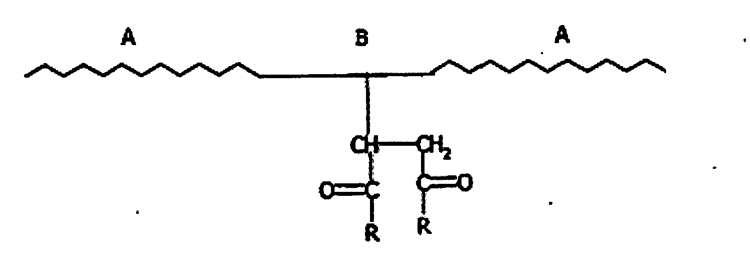
in welcher R eine Gruppe CH₃-O-(CH₂-CH₂-O)ₙ mit mittlerer molarer Masse gleich 2.000, das heißt n = 45, darstellt und das eine durch Gelchromatographie gemessene mittlere molare Masse von 50.000 Dalton hat.

24. Verwendung einer hydrophilen Klebstoffzusammensetzung nach einem der Ansprüche 1 bis 23 zur Herstellung von Produkten, die vorgesehen sind für medizinische, dermatologische, kosmetische oder pharmazeutische Zwecke.

25. Verwendung einer hydrophilen Klebstoffzusammensetzung nach einem der Ansprüche 1 bis 23 zur Herstellung eines Produkts zur Anwendung auf der Haut, einer Wunde oder die Schleimhäute.

26. Verwendung einer hydrophilen Klebstoffzusammensetzung nach einem der Ansprüche 24 oder 25, **dadurch gekennzeichnet, daß** das Produkt ein Pflaster für die Behandlung oder den Schutz einer Wunde, einer Blase, von Verbrennungen oder von Dermis-Epidermis-Oberflächenverletzungen ist, ein Pflaster zur Abgabe von Wirkstoffen auf topischem oder systemischem Wege, ein Produkt zur Pflege, zur Reinigung oder zum Schutz der Haut oder der Schleimhäute, eine Elektrode oder ein Produkt für Stomia.
